# FASCICULE DE BREVET EUROPEEN

(11) **EP 1 486 193 B1**
(45) Date de publication et mention de la délivrance du brevet: **01.09.2010**
(21) Numéro de dépôt: 03705387.3
(22) Date de dépôt: 21.02.2003
(51) Int. Cl.: A61Q 19/00, A61K 8/06, A61K 8/73, A61K 8/891

(54) **PROCEDE DE STABILISATION D'UNE COMPOSITION COSMETIQUE CONTENANT DE L'HUILE DE SILICONE**
VERFAHREN ZUR STABILISIERUNG EINER SILIKONÖL ENTHALTENDEN KOSMETISCHEN ZUSAMMENSETZUNG
METHOD OF STABILIZING SILICONE OIL-CONTAINING COSMETIC COMPOSITION

(30) Priorité: 27.02.2002 JP 2002051390
(43) Date de publication de la demande: 15.12.2004
(73) Titulaire: Hakuto Co., Ltd, Shinjuku-ku, Tokyo 160-8910 (JP)
(72) Inventeur: NOHATA, Yasuhiro,c/o Yokkaichi Laboratory, HAKUTO, Mie 510-0007 (JP); KUROMIYA, Tomomi, c/o Yokkaichi Laboratory, HAKUTO, Mie 510-0007 (JP)
(74) Mandataire: Pautex Schneider, Nicole Véronique
(86) Numéro de dépôt international: PCT/JP2003/001952
(87) Numéro de publication internationale: WO 2003/072070

(56) Documents cités:
- EP-A1- 0 379 999
- EP-A1- 0 524 859
- EP-A1- 0 569 591
- GB-A- 2 164 558
- JP-A- 2001 233 754
- JP-A- 2002 053 426
- JP-A- 2002 060 314
- US-A- 4 788 006

## Description

### Domaine technique de l'invention

La présente invention concerne un procédé, de stabilisation d'une composition cosmétique émulsifiée en mélange avec une huile de silicone, qui est capable d'empêcher une augmentation de la viscosité de produits cosmétiques et une séparation de l'huile de silicone de celle-ci, lors d'un stockage à long terme.

### Art antérieur

Une huile de silicone telle que le diméthylpolysiloxane a été mélangée avec des produits cosmétiques tels que des crèmes, des lotions laiteuses, des lotions et de gelées, parce qu'elle est chimiquement stable, par conséquent moins réactive de façon à être sans danger, et elle est sans odeur et moins collante, elle a une faible tension superficielle avec une bonne capacité d'étalement, et possède un toucher lisse. De plus, ces dernières années, une huile de silicone a été mélangée dans des crèmes solaires, des lotions laiteuses, des gelées, etc., en tirant partie qu'un film hermétique à l'eau d'huile de silicone est efficace pour protéger la peau.

Dans de nombreux cas, une huile de silicone a été mélangée dans des produits cosmétiques sous forme d'une émulsion huile dans eau (H/E) ou d'une émulsion eau dans huile (E/H). L'émulsion huile dans eau (H/E) ou l'émulsion eau dans huile (E/H) est habituellement préparée par addition de composants lipophiles contenant une huile de silicone à des composants hydrophiles en présence d'un tensioactif, ou par addition de composants hydrophiles à des composants lipophiles contenant une huile de silicone en présence d'un tensioactif, et par agitation vigoureuse du mélange résultant. Toutefois, étant donné que l'huile de silicone est hydrophobe et que la tension superficielle de celle-ci est faible, elle se sépare de l'eau et flotte à la surface de l'émulsion, pendant que l'émulsion est stockée pendant une durée prolongée, dégradant de ce fait non seulement l'aspect extérieur des produits, mais aussi l'effet de protection de la peau de l'huile de silicone. Pour surmonter ces problèmes, il a été réalisé des procédés d'amélioration de la stabilité de l'émulsion, tels qu'un procédé consistant à mettre des billes d'agitation dans un récipient des produits cosmétiques et à agiter les billes d'agitation avant emploi, un procédé d'augmentation de la teneur en tensioactif, et un procédé d'utilisation d'une émulsifiant spécifique. Toutefois, le procédé consistant à mettre des billes d'agitation dans le récipient des produits cosmétique n'est pas préférable du point de vue pratique, étant donné que les produits cosmétiques ne peuvent pas être immédiatement utilisés. Les produits cosmétiques doivent être dispersés avec les billes d'agitation par une agitation suffisante du récipient avant emploi. D'autre part, avec le procédé d'augmentation de la teneur en tensioactif, la qualité et les effets des produits cosmétiques peuvent être détériorés, parce que la peau peut être plus irritée par les produits cosmétiques, les produits cosmétiques peuvent être collants ou se décoller facilement par l'eau, ou ne sont guère étalés, en raison de l'augmentation de la quantité du tensioactif mélangé. De plus, divers autres procédés ont été proposés pour améliorer la stabilité de l'émulsion de silicone.

Des exemples de ces procédés englobent un procédé d'utilisation de particules de silicone réticulées ayant un réseau tridimensionnel avec une huile de silicone (demandes de brevet japonais ouvertes à l'inspection publique (JP-A N° 1-165509, 1-190757, 1-207354, 2-243612 et 5-262987), un procédé d'utilisation d'une huile de silicone, d'une silicone réticulée et d'un tensioactif à base d'une silicone modifiée (JP-A N° 3-79669), un procédé d'utilisation d'une huile de silicone de faible viscosité et d'une huile de silicone qui est solide à la température ambiante (JP-A N° 3-271211) et un procédé d'utilisation d'une huile de silicone de faible masse moléculaire et d'une huile de silicone de masse moléculaire élevée ou d'une huile de silicone caoutchouteuse (publication nationale de la demande de brevet japonais N° 6-502646 et JP-A N° 7-330537). De plus, une huile de silicone ayant une mauvaise.compatibilité avec des agents huileux ou avec des parfums solubles dans une huile habituellement utilisés pour les produits cosmétique, tels que des esters d'acides gras obtenus d'huiles animales/végétales naturelles ou synthétiques de sorte qu'ils présentent une mauvaise stabilité de l'émulsion. C'est pourquoi, il a été aussi proposé un procédé d'amélioration de la compatibilité d'une huile de silicone avec d'autres agents huileux par addition de composés de silicone qui sont obtenus par la réaction d'addition des composés de silicone substitués par des groupes fluoroalkyle à un organohydrogénopolysiloxane (JP-A N° 7-197055).

Des exemples de l'huile de silicone utilisée dans ces inventions englobent une silicone modifiée par un alcoxy supérieur tel que le diméthylpolysiloxane, le méthylphénylpolysiloxane et le méthylhydrogénopolysiloxane, l'octaméthylcyclotétrasiloxane, le décaméthylcyclopentasiloxane, le dodécaméthylcyclohexasiloxane, le tétraméthyltétrahydrogénocyclotétrasiloxane et la stéaroxysilicone, et les huiles à base de silicone telles que la silicone modifiée par des acides gras supérieurs, les résines de silicone et les caoutchoucs de silicone, les huiles de silicone, etc.

De plus, il existe des produits de maquillage de base tels qu'une poudre pour le visage et des crèmes de fond de teint pour conditionner la couleur et la texture de la peau, des produits de maquillage précis tels que le rouge à lèvre, le fard à joues, le crayon pour les yeux, le mascara, l'ombre à oeil et le crayon à sourcil pour accentuer une coloration partielle, et des produits cosmétiques préparés par mélange d'une poudre fine et/ou d'un pigment et d'une huile de silicone avec une lotion et une lotion laiteuse telle qu'une lotion carminée pour prévenir une usure du maquillage et pour renforcer la sensation lisse, et analogues. Etant donné qu'il est difficile de permettre à une huile de silicone et à une poudre fine et/ou un pigment de se disperser de façon homogène l'un dans l'autre sur une période prolongée, ces produits cosmétiques ont été vendus sous forme de produits à plusieurs phases qui doivent être agités avant emploi pour disperser les poudres les unes dans les autres. Pour résoudre ce problème, un procédé d'augmentation de la viscosité pour empêcher une séparation des poudres a aussi été envisagé, mais lorsque la viscosité augmente, la sensation fraîche est perdue de sorte que ce procédé n'est pas préférable. Par conséquent, il a été proposé un procédé d'utilisation d'un pigment qui contient des poudres ajustées à un diamètre de particule identique (JP-A N° 11-199422), un procédé d'utilisation de poudres organiques traitées en surface pour améliorer une nouvelle dispersion (JP-A N° 11-292738), un procédé d'addition de sels solubles dans l'eau ayant une fonction de coagulation modérée des poudres JP-A N° 58-177910), etc. Toutefois, il n'a pas été fourni un quelconque produit cosmétique mélangé avec des poudres et une huile de silicone, qui présente une sensation rafraîchissante lors de l'utilisation et qui conserve un état stable dispersé de façon homogène.

Des exemples de poudres utilisées dans ces invention englobent les pigment de verni, des pigments organiques, des pigments colorants, des pigments blancs, des pigments diluants, des pigments de lustre nacrés et des poudres de polymère (pigments).

De plus, on connaît aussi un procédé d'addition d'un polysaccharide constitué de monosaccharides tels que le fucose, le glucose, l'acide glucuronique et la rhamnose, qui est produit par culture d'une souche de micro-organisme Alcaligenes latus B-16, pour des produits cosmétiques pour disperser des substances incompatibles les unes avec les autres, telles que l'eau, une huile, une poudre, etc., et de conserver l'état dispersé de celles-ci sur une durée prolongée, afin de fournir une stabilité élevée dans l'émulsification et la dispersion des produits cosmétiques (JP-A N° 2001-226250).

De plus, on connaît aussi un procédé d'addition d'un polysaccharide constitué de monosaccharides tels que le fucose, le glucose, l'acide glucuronique et la rhamnose, qui est produit par culture d'une souche de micro-organisme Alcaligenes latus B-16, à des médicaments pour la peau pour une utilisation externe, qui contient l'huile de silicone décrite ci-dessus et des agents huileux à base de fluor tels que le perfluoropolyéther, le perfluorodécane et le perfluorooctane, avec des composants de soin de la peau tels qu'un agent de conservation de l'humidité, un astringent, un agent de blanchiment, un agent anti-inflammatoire, un agent de prévention des UV et un agent activant les cellules, etc. ou des composants de blanchiment y compris des produits chimiques tels que les acides ascorbiques et les composants animaux et végétaux tels que l'huile de réglisse pour stabiliser les médicaments pour la peau. Avec ce procédé, des solutions aqueuses, des lotions laiteuses, des dispersions en suspension, des crèmes, des pâtes, des mousses et des gelées et, plus spécifiquement, des produits cosmétiques de base tels que des crème de nettoyage de la peau, des crèmes de nettoyage, des lotions, des liquides de beauté, des pansements, des crèmes de massage, des crèmes, des lotions laiteuses, des crèmes pour les lèvres, des produits cosmétiques de maquillage de base tels qu'un fard et une poudre pour le visage ; des produits cosmétiques précis tels qu'un rouge à lèvre, le rouge pour les joues, une ombre pour les yeux, un crayon pour les yeux et le mascara, et des produits cosmétiques pour le corps tels que les vernis à ongle, les produits cosmétiques solaires, un anti-transpirant, les agents de blocage des odeurs, des agents désodorisants, un agent épilatoire, les agents repoussants les insectes et les agents pour le bain, peuvent être stabilisés (JP-A- N° 2002-60314 et 2002-29929).

De plus, on connaît aussi un procédé d'addition d'un polysaccharide constitué de fucose, de glucose, d'acide glucuronique et de rhamnose en tant que monosaccharides, qui est produit par culture d'une souche de micro-organisme Alcaligenes latus B-16, à une composition cosmétique aqueuse mélangée avec des pigments fonctionnels, qui contient des huiles de silicones telles que le méthylpolysiloxane et le méthylphénylpolysiloxane, des agents huileux y compris les huiles et les matières grasses telles que l'huile d'olive, l'huile de camélia, l'huile de noix de macadam, et l'huile de ricin, les cires telles que la cire de carnabée, la cire de candélia, l'huile de jojoba, la cire d'abeille et la lanoline, les hydrocarbures tels que la paraffine liquide, la paraffine, la vaseline, la sérésine et le squalane, les acides gras supérieurs tels que l'acide laurique, l'acide myristique, l'acide palmitique, l'acide stéarique et l'acide isostéarique, les alcools supérieurs tels que l'alcool cétylique, l'alcool stéarylique et l'alcool isostéarylique, les esters tels que le myristate d'isopropyle, le myristate de 2-octyldodécyle, le 2-éthylhexanoate de cétyle, le malate de diisostéaryle, ainsi que les émulsifiants, les agents de conservation de l'humidité, les astringents, les agents de blanchiment, les agents anti-UV, les agents anti-inflammatoires et les agents activant les cellules de la peau, en fonction du but des produits cosmétiques. Avec ce procédé, les produits cosmétiques y compris les produits de maquillage de base tels que les fonds de teint, les produits de maquillage précis tels que le rouge à lèvre, le rouge pour les joues, le crayon pour les yeux, le mascara, l'ombre pour les yeux et le crayon pour les sourcils, ainsi qu'une lotion carminée, une lotion et une lotion laiteuse, sont stabilisés (JP-A N° 2002-53426).

Toutefois, lorsque des poudres sont contenues dans la composition cosmétique mélangée avec l'huile de silicone, on n'a pas obtenu une composition cosmétique satisfaisante qui présente une bonne compatibilité avec d'autres composants lipophiles et d'autres composants hydrophiles sur une durée prolongée sans compromettre la stabilité dans les poudres, et qui présente aussi une excellente stabilité sans aucune séparation de l'huile de silicone des produits cosmétiques résultants, et sans aucune détérioration de la sensation rafraîchissante à l'emploi, par les procédés décrits ci-dessus.

Dans les circonstances décrites ci-dessus, le but de la présente invention est de fournir un procédé, de stabilisation d'une composition cosmétique émulsifiée mélangée avec une huile de silicone, qui est capable d'empêcher une augmentation de la viscosité des produits cosmétiques, et une séparation de l'huile de silicone de ceux-ci, lorsqu'ils sont stockés pendant une durée prolongée, et de maintenir la sensation légère de ceux-ci dépourvue d'une sensation collante à l'emploi.

### Description de l'invention

Les présents inventeurs ont mené des études poussées pour fournir des procédés de stabilisation d'une dispersion d'une huile de silicone dans des compositions cosmétiques mélangées avec une huile de silicone. Ils ont ainsi découvert qu'en mélangeant un polysaccharide spécifique dans les compositions cosmétiques, une dispersion d'une huile de silicone dans de l'eau peut être stabilisée pendant une durée prolongée, et qu'en mélangeant le polysaccharide spécifique dans un rapport spécifique à l'huile de silicone, cet effet est notablement amélioré, et ils ont réalisé la présente invention.

A savoir, l'invention selon la revendication 1 fournit un procédé de stabilisation d'une composition cosmétique émulsifiée mélangée avec une huile de silicone comprenant des étapes de mélange d'un polysaccharide constitué au moins de fucose, de glucose, d'acide glucuronique et de rhamnose en tant que monosaccharides constitutifs dans la composition cosmétique émulsifiée et de mélange d'un tensioactif dans la composition cosmétique émulsifiée de sorte que la teneur dudit tensioactif s'échelonne de 0,1 à 20 % en poids, par rapport à l'huile de silicone.

En mélangeant le polysaccharide décrit ci-dessus, on peut obtenir la composition cosmétique émulsifiée mélangée avec une huile de silicone, qui peut conserver un état de dispersion stabilisé pendant une durée prolongée avec une teneur en polysaccharide plus faible, par rapport au cas où un polysaccharide classique est utilisé, et qui présente une sensation rafraîchissante des produits cosmétiques à l'emploi.

De plus, en mélangeant 5 à 20 % en poids de tensioactif, par rapport à l'huile de silicone, on stabilise mieux l'émulsion d'huile de silicone, et on peut obtenir une composition cosmétique entraînant peu d'irritation de la peau et ne présentant pas une sensation collante.

L'invention selon la revendication 2 fournit le procédé de stabilisation d'une composition cosmétique émulsifiée mélangée avec une huile de silicone selon la revendication 1, dans lequel ledit polysaccharide est un produit d'un micro-organisme de la souche Alcaligenes latus B-16.

On peut obtenir plus efficacement le polysaccharide en tant que produit en utilisant le micro-organisme de la souche Alcaligenes latus B-16.

L'invention selon la revendication 3 fournit le procédé de stabilisation d'une composition cosmétique émulsifiée mélangée avec une huile de silicone selon la revendication 1 ou 2, dans lequel ledit tensioactif est au moins un composé parmi un ester d'acide gras de saccharose, un ester d'acide gras de glycérine, un ester d'acide gras de polyglycérine, la lécithine et les dérivés de ceux-ci.

On peut mieux stabiliser la composition cosmétique émulsifiée mélangée avec une huile de silicone en utilisant au moins un composé parmi un ester d'acide gras de saccharose, un ester d'acide gras de glycérine et un ester d'acide gras de polyglycérine en tant que tensioactif.

L'invention selon la revendication 4 fournit le procédé de stabilisation d'une composition cosmétique émulsifiée mélangée avec une huile de silicone selon l'une quelconque des revendications 1 à 3, dans lequel la teneur du polysaccharide est de 0,1 à 5 % en poids, convertie en fraction solide séchée, par rapport à l'huile de silicone.

En mélangeant 0,1 à 5 % en poids (convertie en fraction solide séchée) du polysaccharide par rapport à l'huile de silicone, on stabilisé l'émulsion d'huile de silicone et on peut obtenir une composition cosmétique présentant une excellente sensation à l'emploi.

### Le meilleur mode de réalisation pour mettre en oeuvre l'invention

Une composition cosmétique mélangée avec une huile de silicone selon la présente invention est la composition cosmétique dans laquelle une huile de silicone est dispersée de façon stable en présence d'un polysaccharide spécifique. L'état de dispersion de l'huile de silicone dans la composition cosmétique n'est pas particulièrement limité, tant que l'huile de silicone y est dispersée de façon stable, et des exemples de celui-ci englobent une émulsion dispersée, une émulsion huile dans eau (appelée "type H/E" dans la suite) et une émulsion eau dans huile (appelée '"type E/H" dans la suite), ainsi qu'une émulsion composite E/H/E ou H/E/H et une composition cosmétique mélangée avec une huile de silicone de type microcapcule.

La composition cosmétique de la présente invention est sous forme d'une solution, d'une crème, d'une pâte, d'une gelée, etc. Des exemples de celles-ci englobent les produits cosmétiques de base tels que les crèmes de nettoyage du visage, les crèmes de nettoyage, une lotion, un pansement, un crème de massage, une lotion laiteuse conservant l'humidité, une crème conservant l'humidité, une crème pour les lèvres, etc., les produits cosmétiques de finition, tels que le fond de teint, l'ombre pour les yeux, le crayon pour les yeux, le mascara, le rouge à lèvre, etc., les produits cosmétiques capillaires tels que la gelée pour les chevaux, la crème capillaire, le shampoing, un produit de rinçage, un traitement capillaire, un produit de conditionnement capillaire, un liquide capillaire, une lotion fixante, etc., les produits cosmétiques de type parfum, tels que le parfum (parfum et produit parfumé), l'eau de parfum (eau de cologne), l'eau de toiletté (parfum de toilette, et produit parfumé de toilette), l'eau de cologne (Cologne, Cologne frais), etc.,, les produits cosmétiques contre les coups de soleil tels qu'une gelée solaire, une crème solaire, une lotion laiteuse solaire, etc.

La présente invention est **caractérisée en ce qu**'un polysaccharide spécifique est mélangé avec la composition cosmétique mélangée avec une huile de silicone, et avec un tensioactif. La composition cosmétique mélangée avec une huile de silicone est fabriquée par un procédé classique dans lequel, en présence d'un tensioactif, un composant lipophile contenant une huile de silicone est ajouté à un composé hydrophile, ou un composant hydrophile est ajouté à un composant lipophile contenant une huile de silicone, et le mélange résultant est agité. Selon la présente invention, le polysaccharide est mélangé pendant la fabrication de la composition cosmétique mélangée avec l'huile de silicone.

L'huile de silicone à utiliser dans la présente invention est un polymère ayant une liaison siloxane représentée par la formule générale (1) en tant que chaîne principale de celui-ci.

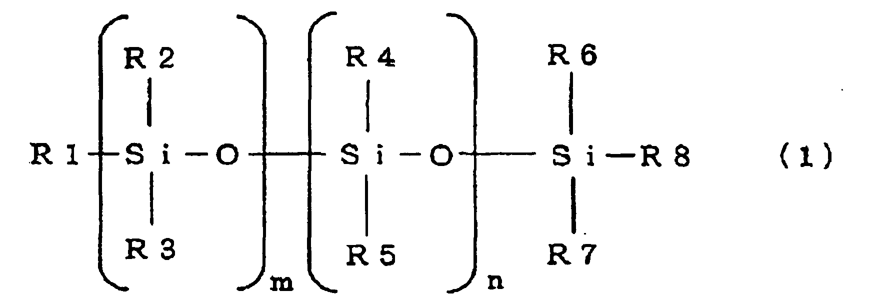

Dans la formule générale, R1, R2, R3, R6, R7 et R8 représentent respectivement un atome d'hydrogène, un groupe hydroxyle, un groupe alkyle comportant 1 à 22 atomes de carbone qui peut comprendre un groupe fluoré, un résidu d'alcool comportant 1 à 22 atomes de carbone, un résidu d'acide carboxylique comportant 1 à 22 atomes de carbone ou un groupe aryle comportant 6 à 24 atomes de carbone, et R4 et R5 représentent respectivement un atome d'hydrogène, un groupe hydroxyle, un groupe alkyle comportant 1 à 22 atomes de carbone qui peut comprendre un groupe fluoré, un résidu d'alcool comportant 1 à 22 atomes de carbone, un résidu d'acide carboxylique comportant 1 à 22 atomes de carbone, un groupe aryle comportant 6 à 24 atomes de carbone, un polymère d'oxyde d'alkylène comportant 2 à 4 atomes de carbone ou un résidu de polymère d'oxyde d'alkylène dans lequel une extrémité du polymère d'oxyde d'alkylène comportant 2 à 4 atomes de carbone est estérifiée avec un acide gras comportant 2 à 22 atomes de carbone, ou éthérifié avec un alcool supérieur comportant 2 à 22 atomes de carbone ou un groupe aryle comportant 6 à 24 atomes de carbone.

Les lettres m et n sont des nombres entiers de 2 ou plus.

Des exemples du groupe alkyle comportant 1 à 22 atomes de carbone qui peuvent contenir un groupe fluoré englobent un groupe méthyle, un groupe éthyle, un groupe propyle, un groupe isopropyle, un groupe n-butyle, un groupe isobutyle, un groupe sec-butyle, un groupe tert-butyle, un groupe pentyle, un groupe hexyle, un groupe octyle, un groupe nonyle, un groupe décyle, un groupe dodécyle, un groupe tétradécyle, un groupe hexadécyle, un groupe stéaryle, un groupe oléyle et un groupe béhényle, etc., et l'un au moins d'entre eux est utilisé.

Des exemples du résidu d'alcool comportant 1 à 22 atomes de carbone englobent un groupe méthoxy, un groupe éthoxy, un groupe propoxy, un groupe isopropoxy, un groupe butoxy, un groupe isobutoxy, un groupe sec-butoxy, un groupe tert-butoxy, un groupe pentoxy, un résidu de décanol, un résidu de dodécanol, un résidu d'alcool de palm, un résidu d'alcool stéarylique, un résidu d'alcool oléylique et un résidu d'alcool béhénylique, etc., et l'un au moins d'entre eux est utilisé.

Des exemples du résidu d'acide carboxylique comportant 1 à 22 atomes de carbone englobent un groupe acétyle, un résidu d'acide propionique, un résidu d'acide butanoïque, un résidu d'acide pentanoïque, un résidu d'acide hexanoïque, un résidu d'acide octanoïque, un résidu d'acide décanoïque, un résidu d'acide dodécanoique, un résidu d'acide stéarique, un résidu d'acide oléique et un résidu d'acide béhénique, etc., et l'un au moins d'entre eux est utilisé.

Des exemples du groupe aryle comportant 6 à 24 atomes de carbone englobent un groupe phényle, un groupe benzyle, un groupe éthyphényle, un groupe octylphényle, un groupe nonylphényle, et un groupe stéarylphényle, etc., et l'un au moins d'entre eux est utilisé.

Des exemples du polymère d'oxyde d'alkylène comportant 2 à 4 atomes de carbone englobent un homopolymère d'oxyde d'éthylène, d'oxyde de propylène et d'oxyde de butylène, ou un polymère d'addition d'un mélange d'au moins deux d'entre eux, et le degré de polymérisation (n) de celui-ci est habituellement de 1 à 100.

Des exemples de l'huile de silicone englobent le diméthylpolysiloxane, l'éthylméthylpolysiloxane, le diéthylpolysiloxane, le méthylhydrogénopolysiloxane, le méthylphénylpolysiloxane, un organopolysiloxane modifié par un polyéther tel qu'un copolymère diméthylsiloxaneméthyl(polyoxyéthylène)siloxane et un copolymère diméthylsiloxane-méthyl(polyoxyéthylènepolyoxypropylène)siloxane, un diméthylpolysiloxane cyclique tel qu'un copolymère diméthylsiloxanealcoxy(comportant 4 à 12 atomes de carbone)-méthylsiloxane, l'octaméthylcyclotétrasiloxane, le décaméthylcyclopentasiloxane, le dodécaméthylcyclohexasiloxane, un organosiloxane modifié par du fluor tel qu'un copolymère fluorométhylsiloxanediméthylsiloxane, un organopolysiloxane modifié par un fluoroalkylpolysiloxane tel qu'un copolymère fluorométhylsiloxane-polyoxyéthylèneméthylsiloxane et un copolymère fluorométhylsiloxane-polyoxyéthylènepolyoxypropylèneméthylsiloxane, un organopolysiloxane à extrémité ou à chaîne latérale modifiée tel qu'une substance modifiée par un diméthylpolysiloxane dans laquelle un groupe hydroxyle est introduit à une extrémité de celle-ci, et un copolymère hydroxyméthylsiloxane-diméthylpolysiloxane dans lequel un groupe hydroxyle est partiellement introduit dans une chaîne latérale, et un aminoorganopolysiloxane modifié tel qu'un polymère diméthylaminobutylméthylsiloxane-diméthylsiloxane ayant un groupe dialkylaminoalkyle dans une chaîne latérale de celui-ci, etc. Habituellement, l'huile de silicone ayant une viscosité de 100 000 (mPa.s ; 25°C) ou moins est choisie comme composant de la composition cosmétique. La teneur de cette huile de silicone s'échelonne de 0,1 à 80 % en poids (par rapport à la quantité totale de la composition cosmétique), et la teneur préférable de celle-ci s'échelonne de 0,5 à 50 % en poids (dans la suite, "% en poids" sera appelé %).

Le polysaccharide à utiliser dans la présente invention est le polysaccharide constitué d'un moins du fucose, du glucose, de l'acide glucuronique et de la rhamnose en tant que monosaccharides constitutifs et, comme représenté par la formule (2) suivante, il a de préférence des chaînes principales avec un construction de répétition constituée de fucose, d'acide glucuronique et de rhamnose, où un fucose diverge d'un glucose dans la chaîne principale.

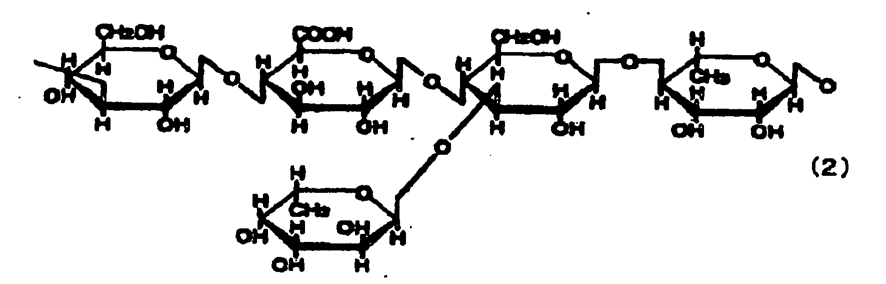

Le polysaccharide représenté par la formule (2) peut être obtenu, par exemple, sous forme d'un produit d'un micro-organisme de la souche Alcaligenes latus B-16 (FERM BP-2015). Le micro-organisme de la souche Alcaligenes latus B-16 est cultivé par un procédé de culture de microbes habituel et, après la culture, des solvants organiques tels que l'acétone, l'éthanol et l'alcool isopropylique, sont ajoutés au liquide de culture. Le polysaccharide précipite ainsi sous forme d'une substance insoluble. La précipitation du polysaccharide est séparé, afin de donner un polysaccharide.

Généralement, les micro-organismes produisent deux types ou plus de polysaccharides. D'autres types de polysaccharide que le polysaccharide de la présente invention peuvent être incorporés, à condition qu'ils n'altèrent pas les effets de la présente invention. Par exemple, il a été prouvé que les polysaccharides produits par un micro-organisme de la souche Alcaligenes latus B-16 comprennent au moins deux types de polysaccharides, et le rapport molaire des monosaccharides constitutifs des polysaccharides qui sont séparés du liquide de culture est :
fucose : glucose : acide glucuronique : rhamnose = 1 : (0,5 à 4) : (0,5 à 2) : (0,5 à 2).

Lorsque deux types de polysaccharides sont séparés l'un de l'autre, un type de polysaccharide est un polysaccharide ayant une construction dans laquelle un fucose diverge d'un glucose dans les chaînes principales avec une construction répétée, qui est constituée de glucose, d'acide glucuronique et de rhamnose, comme le montre la formule (2), et un autre type de polysaccharide est un polysaccharide duquel un motif de répétition est constitué de fucose et de mannose. Le premier est le polysaccharide de la présente invention, a un rapport de constitution du fucose, glucose, acide glucuronique et rhamnose qui est de 1 : 2 : 1 : 1, et qui est un composant de masse moléculaire élevée ayant une masse moléculaire d'environ 10⁹ [voir le Japan Agricultural Chemistry Society, 1998 the Year Large Meeting, résumé, page 371]. Le deuxième est un polysaccharide ayant une construction de répétition de fucose et de mannose de 1 : 1, et est un composant de faible masse moléculaire ayant une masse moléculaire de 10³ à 10⁷ [voir Y. Nohata, J. Azuma, R. Kurane, Carbohydrate Research 293, (1996) 213 à 222]. Ce composant de faible masse moléculaire ne se situe pas dans le cadre du polysaccharide de la présente invention, mais il n'altère par l'effet de stabilité de la présente invention, de sorte qu'il peut être contenu dans la composition cosmétique mélangée avec une huile de silicone.

Le polysaccharide est mélangé dans la composition cosmétique en une proportion de 0,1 à 5 %, de préférence de 0,3 à 3 %, et mieux encore de 0,5 à 2 %, convertie en la fraction solide sèche, par rapport à quantité d'huile de silicone. On détermine la quantité mélangée en considérant l'effet de stabilisation de la composition cosmétique mélangée avec l'huile de silicone, et on ne peut pas obtenir un effet suffisant lorsque la quantité mélangée est inférieure à 0,1 % ou supérieure à 5 %.

Il est nécessaire d'utiliser le tensioactif pour former une émulsion plus stable de l'huile de silicone hydrophobe et des composants hydrophiles. Des exemples des tensioactifs à utiliser dans la présente invention englobent les tensioactifs non ioniques, les tensioactifs anioniques, les tensioactifs cationiques et les tensioactifs amphotères.

Des exemples de tensioactifs non ioniques englobent les éthers alkyliques polyalcoxylés, les éthers alkylphényliques polyalcoxylés, les esters d'acides gras polyalcoxylés, les esters d'acides gras de sorbitan polyalcoxylés, les esters d'acides gras de sorbitan, les esters d'acide gras de glycérine polyalcoxylés, les esters d'acide gras de polyglycérine, les huiles de ricin hydrogénées polyalcoxylés, les esters d'acides gras de saccharose, les alkylamines polyalcoxylées, les copolymères séquencés oxyde d'éthylène-oxyde de propylène, etc.

Le polyoxyalkykène dans les tensioactifs non iniques est constitué d'au moins un polyoxyéthylène (appelé "POE" dans la suite), un polyoxypropylène (appelé "POP" dans la suite), et de polyoxybutylène (appelé "POB" dans la suite). Le nombre de moles de polymérisation de chacun de POE, POP et POB est déterminé arbitrairement, par rapport aux caractéristiques d'émulsion souhaitées du tensioactif, et il est habituellement de 3 à 200. De plus le rapport molaire de polymérisation du POE, POP et POB est déterminé arbitrairement, par rapport aux caractéristiques d'émulsion souhaitées du tensioactif. De préférence, le polyoxyalkylène est constitué de POE et de POP, et un rapport molaire de POE est de 25 % en moles ou plus.

Les éthers alkyliques polyalcoxylés comportant 2 à 4 atomes de carbone sont préparés par addition d'un poly(oxyde d'alkylène) à un alcool saturé ou insaturé, du type à chaîne droite ou de type ramifié, comportant 8 à 30 atomes de carbone. Plus spécifiquement, des exemples des éthers alkyliques polyalcoxylés comportant 2 à 4 atomes de carbone englobent l'octyléther de POE (3 moles), le dodécyléther de POE (5 moles), l'oléyléther de POE (10 moles), le stéaryléther de POE (15 moles), le béhényléther de POE (20 moles), le décyléther de POE (10 moles) et de POP (10 moles), l'isostéaryléther de POE (15 moles) et de POP (2 moles), le cholestanoléther de POE (10 moles), les lanolines hydrogénées de POE (0 mole) et de POP (0 mole), etc.

Les éthers alkylphényliques polyalcoxylés sont préparés par addition de poly(oxyde d'alkylène) à un alkylphénol et à un alcénylphénol de type chaîne droite ou de type ramifié, comportant chacun 1 à 22 atomes de carbone. Plus spécifiquement, des exemples d'éther alkylphényliques polyalcoxylés englobent le méthylphényléther polyéthylé (3 moles), l'octylphényléther de POE (5 moles), le nonylphényléther de POE (10 moles), le dodécylphényléther de POE (15 moles), etc.

Les esters d'acides gras polyalcoxylés sont préparés par addition d'un poly(oxyde d'alkylène) à un acides gras saturé de type chaîne droite ou de type ramifié ou à un acide gras insaturé comportant 8 à 22 atomes de carbone. Plus spécifiquement, des exemples des esters d'acides gras polyalcoxylés englobent l'ester d'acide octanique de POE (3 moles), l'ester d'acide décanique de POE (5 moles), l'ester d'acide dodécanique de POE (10 moles), l'ester d'acide stéarique de POE (15 moles), l'ester d'acide béhénique de POE (20 moles), l'ester d'acide isostéarique de POE (15 moles), l'ester d'acide oléique de POE (15 moles) et de POP (5 moles), etc.

Les esters d'acides gras de sorbitan polyalcoxylés sont préparés par addition de poly(oxyde d'alkylène) à du sorbitol et à un acide gras saturé de type chaîne droite ou de type ramifié ou à un acide gras insaturé comportant 8 à 22 atomes de carbone. Plus spécifiquement, des exemples des esters d'acides gras de sorbitan polyalcoxylés englobent le monolaurate de sorbitan de PEO (5 moles), le trilaurate de sorbitan de PEO (20 moles), le monostéarate de sorbitan de POE (20 moles), le sesquistéarate de sorbitan de POE (20 moles), le tristéarate de sorbitan de POE (20 moles), le monooléate de sorbitan de POE (20 moles), etc.

Les esters d'acides gras de sorbitan sont des esters constitués de sorbitol et d'un acide gras saturé de type chaîne droite ou de type ramifié ou à un acide gras insaturé comportant 8 à 22 atomes de carbone. Plus spécifiquement, des exemples des esters d'acides gras de sorbitan englobent le monolaurate de sorbitan, le monopalmitate de sorbitan, le monostéarate de sorbitan, le monoisostéarate de sorbitan, le monooléate de sorbitan, le sesquioléate de sorbitan, le trioléate de sorbitan, le diglycérolsorbitan d'acide penta-2-éthylhexylique, le diglycérolsorbitan d'acide tétra-2-éthylhexylique, etc.

Les esters d'acides gras de glycérine polyalcoxylés sont des esters d'addition constitués de glycérine, d'un acide gras saturé de type chaîne droite ou de type ramifié ou d'un acide gras insaturé comportant 8 à 22 atomes de carbone et de poly(oxyde d'alkylène). Plus spécifiquement, des exemples des esters d'acides gras de glycérine polyalcoxylés englobent le monolaurate de glycérol de POE (5 moles), le monostéarate de glycérol de POE (10 moles), le distéarate de glycérol de POE (15 moles), le dioléate de glycérol de POE (20 moles) et de POP (5 moles), etc.

Les esters d'acides gras de glycérine sont des esters constitués de glycérine et d'un acide gras saturé de type chaîne droite ou de type ramifié ou à un acide gras insaturé comportant 8 à 22 atomes de carbone. Plus spécifiquement, des exemples des esters d'acides gras de glycérine englobent le monolaurate de glycéryle, le sesquilaurate de glycéryle, le trilaurate de glycéryle, le monostéarat' de glycéryle, le sesquistéarate de glycéryle, le tristéarate de glycéryle, le monooléate de glycéryle, le sesquioléate de glycéryle, le trioléate de glycéryle, le monoglycéride d'acide gras d'huile de graine de coton, le monoétucate de glycéryle, le pyroglutamate d'α,α'-glycéryle, l'ester d'un mélange d'acides gras saturés comportant 8 à 12 atomes de carbone et de glycérine et l'ester de l'acide stéarique, de l'acide malique et de glycérine, etc.

Des exemples d'esters d'acide gras de polyglycérine englobent le polyglycérolester d'acide hydroxystéarique condensé, le polyglycérolester d'acide ricinoléique condensé, etc., et des exemples d'huiles de ricin hydroxylées polyalcoxylées englobent l'huile de ricin de POE (10 moles), l'huile de ricin hydrogénée de POE (15 moles), le monoisostéarate d'huile de ricin hydrogénée de POE (15 moles), le triisostéarate d'huile de ricin hydrogénée de POE (20 moles), le diester monopyroglutamate-monoisostéarate d'huile de ricin hydrogénée de POE (20 moles), le malénate d'huile de ricin hydrogénée de POE (20 moles), etc.

Les esters d'acides gras de saccharose sont des esters constitués de saccharose et d'un acide gras saturé de type chaîne droite ou de type ramifié ou à un acide gras insaturé comportant 8 à 22 atomes de carbone. Plus spécifiquement, des exemples des esters d'acides gras de saccharose englobent le béhénate de saccharose, le stéarate de saccharose, le palmitate de saccharose, le miristate de saccharose, le laurate de saccharose, l'érucate de saccharose, l'oléate de saccharose, etc.

Les alkylamines polyalcoxylées sont préparées par addition de poly(oxyde d'alkylène) à une amine primaire ou secondaire comportant 3 à 22 atomes de carbone et, plus spécifiquement, des exemples de celles-ci englobent la didodécylamine de POE (5 moles), la dodécylamine de diPOE (10) et de POP (3), la distéarylamine de POE (10 moles), la stéarylamine de diPOE (10 moles), l'oléylamine de diPOE (15 moles), la béhénylamine de diPOE (17 moles), etc.

Les copolymères oxyde d'éthylène-oxyde de propylène sont des copolymères obtenus par polymérisation d'oxyde d'éthylène et d'oxyde de propylène dans un rapport molaire de 1:9 à 9:1 de façon à obtenir une masse moléculaire d'environ 500 à 50 000.

De plus, des exemples de tensioactifs anioniques englobent les sels d'acides gras, les sels d'alkylsulfate, les sels d'alcénylsulfate, les sels d'alkylphénylsulfate et les sels d'alcénylphénylsulfate, les sels d'alkylphényléthersulfate polyalcoxylés et les sels d'alcénylphényléthersulfate polyalcoxylés, les sels de sulfosuccinate de (di)alkyle, les sels de N-acylaminé (les acyl-N-méthyltaurines), les sels d'alkylbenzènesulfonate, les sels d'alkylnaphtalènesulfonate, la polycondensation de formaline de sels de naphtalènesulfonate, etc. Des exemples préférables de sels métalliques englobent les sels de sodium, les sels de potassium et les sels d'ammonium.

Les sels d'acides gras sont des sels métalliques d'un acide gras saturé de type à chaîne droite ou de type ramifié ou d'un acide gras insaturé comportant 8 à 30 atomes de carbone et, plus spécifiquement, des exemples de ceux-ci englobent l'octylate de sodium, le décanoate de sodium, le dodécanoate de sodium, le tétradécanate de sodium, le stéarate de sodium, l'isostéarate de sodium, l'oléate de sodium, le linolénate de sodium, l'édétate de sodium, etc.

Les alkylsulfates et les alcénylsulfates sont des alkylsulfates et des alcénylsulfates saturés de type à chaîne droite ou de type ramifié ou insaturés comportant 8 à 30 atomes de carbone et, plus spécifiquement, des exemples de ceux-ci englobent l'octylsulfate de sodium, le décylsulfate de sodium, le dodécylsulfate de sodium, l'alkylsulfate de palm de sodium, le stéarylsulfate de sodium, l'isostéarylsulfate de potassium, l'oléylsulfate d'ammonium, le béhénylsulfate d'ammonium, etc.

Les alkylphényléthersulfates et les alcénylphényléthersulfates sont des sels d'ester sulfurique d'un produit d'addition d'un groupe phényle contenant un groupe alkyle ou un groupe alcényle, qui comporte 1 à 22 atomes de carbone et des chaînes droites ou des chaînes ramifiées, et d'un polyoxylkylèneglycol comportant 2 à 4 atomes de carbone et, plus spécifiquement, des exemples de ceux-ci englobent le tosylsulfate de POE (3 moles) de sodium, l'octylphénylsulfate de POE (5 moles) de sodium, le nonylphénylsulfate de POE (10 moles) de potassium, le décylphénylsulfate de POE (10 moles), l'octadécénylsulfate de POE (15 mols) de potassium, l'isooctadécylsulfate de POE (15 moles) et de POP (5 moles) de potassium.

Des exemples de sels de (di)alkylsulfosuccinates englobent le dioctylsulfosuccinate de sodium, le di-2-éthylhexylsulfosuccinate de sodium, le monolaurylmonoéthanolamidosulfosuccinate de sodium polyéthoxylé, le laurylglycolsulfosuccinate de sodium polypropylé, etc.

Les sels d'acides N-acylaminés sont des acyl-N-méthyltaurines et, plus spécifiquement, des exemples de ceux-ci englobent les sels d'amidosulfonate d'acide aminé gras supérieur tels que la lauroylsarcosine de sodium, la N-myristoyl-N-méthyltaurine de sodium, la méthyltaurine d'acide gras de coprah de sodium, la laurylméthyltaurine de sodium, etc., et un glutamate de N-acyle tel que le glutamate de N-lauryle monosodique, le glutamate N-stéaroyle disodique, le glutamate de N-myristyle L-monosodique, etc. Des exemples de sels alkylbenzènesulfonate englobent le dodécylbenzènesulfonate de sodium, le dodécybenzènesulfonate de potassium, le dodécylbenzènesulfonate d'ammonium, etc. Ces substances peuvent être utilisées seules ou en combinaison de deux d'entre elles ou plus.

Des exemples de tensioactifs cationiques englobent les acides aminés, les sels d'alklamine, les sels d'ammonium quaternaires, les sels de pyridium, etc.

Des exemples d'acides aminés englobent la lécithine de jaune d'oeuf ou de soja, ou un dérivé de lécithine tel que la lécithine hydrogénée, l'hydroxyde de lécithine, etc.

Les sels d'alkylamine sont des sels d'amine primaire ou secondaire comportant 3 à 22 atomes de carbone et d'un acide carboxylique comportant 1 à 22 atomes de carbone ou d'un acide minéral. Et des exemples de ceux-ci englobent le sel d'acétate de dodécylamine, le sel de chlorhydrate de dodécylamine, le sel de stéarate de dodécylaline, le sel de stéarate de diméthylamine, etc.

Les sels d'ammonium quaternaires sont des sels d'amine quaternaire comportant 3 à 22 atomes de carbone, et d'un acide carboxylique comportant 1 à 22 atomes de carbone ou d'un acide minéral. Des exemples de ceux-ci englobent le chlorure de stéaryltriméthylammonium, le chlorure de lauryltriméthylammonium, le chlorure de distéaryldiméthylammonium, le chlorure de benzalconium, le chlorure de benzétonium, le sel bromure de palme-alkyl(de 10 à 14 atomes de carbone)isoquinolinium, le sel chlorure de dodécylimidazonium.

Des exemples de sels de pyridinium englobent le chlorure de poly(N,N-diméthyl-3,5-méthylènepipéridinium), le chlorure de cétylpyridinium, etc.

D'autres exemples de tensioactifs cationiques englobent les oxydes d'amine, tels que l'oxyde de dodécyldiméthylamine, un polymère cationique tel qu'un copolymère acide acrylique-acide β-N,N-diméthyl-N-éthylaminoacétique-vinylpyrrolidone, etc.

Des exemples de tensioactifs amphotères englobent les bétaïnes, les phosphobétaïnes, les sulfobétaïnes, les glycinebétaïnes, les bétaïnes d'imidazolium, les oxydes d'amine. Plus spécifiquement, des exemples de bétaïnes englobent l'acétate de dodécyldiméthylamino-bétaine, l'acétate de stéaryldiméthylamino-bétaïne, l'acétate d'amidopropyldiméthylamino-bétaine d'acide décanique, etc. Des exemples de phosphobétaïnes englobent le 2-(diméthyldodécylammonio)propiophosphate, le 2-(diméthyldodécylammonio)-2-hydroxypropiophosphate, etc. Des exemples de sulfobétaines englobent le dodécyldiméthyléthosulfate d'ammonium, etc. Des exemples de glycinebétaine englobent la dodécyl-di(aminoéthyl)glycine, etc. Des exemples de bétaïne d'imidazolium englobent la 2-undécyl-N,N,N-(hydroxyéthylcarboxyméthyl)-2-imidazoline de sodium, l'hydroxyde de 2-coprah-2-imidazolinium, le sel de sodium de 1-carboxyéthoxy-2, la bétaïne de 2-heptadécyl-N-carboxyméthyl-N-hydroxyéthylimidazolinium, etc.

Des exemples préférés du tensioactif englobent un ester d'acide gras de saccharose, un ester d'acide gras de glycérine, un ester d'acide gras de polyglycéryle, la lécithine et un dérivé de celle-ci, et les exemples particulièrement préférés englobent le stéarate de saccharose, le palmitate de saccharose, le myristate de saccharose, le laurate de saccharose, l'éruciate de saccharose, l'oléate de saccharose, le monostéarate de glycéryle, l'oléate de glycéryle, le polyglycérineester d'acide hydroxystéarique condensé, le polyglycérylester de ricinoléate condensé, la lécithine, la lécithine hydrogénée et l'hydroxyde de lécithine. Ces substances peuvent être utilisées seules ou en combinaison de deux d'entre elles ou plus.

La teneur en tensioactif est convenablement choisie en fonction du type de produits cosmétiques, du type d'huile de silicone et de la quantité mélangée de celle-ci, et elle n'est pas déterminée au hasard. Toutefois, la teneur en tensioactif doit être de 0,1 à 20 % en poids, de préférence de 0,5 à 20 % en poids, par rapport à la teneur en huile de silicone. Dans une composition cosmétique classique, la teneur en tensioactif est de 50 à 100 % en poids, par rapport à la teneur en huile de silicone, ou de 5 à 20 % en poids par rapport à la quantité totale de la composition. En revanche, en utilisant le polysaccharide selon la présente invention, on peut obtenir une stabilité de dispersion plus élevée de l'huile de silicone avec une teneur plus faible en tensioactif, par rapport à la composition cosmétique classique. En outre, on peut obtenir des produits cosmétiques fournissant une sensation légère et fraîche sans sensation collante.

Le procédé de production de la composition cosmétique mélangée avec l'huile de silicone selon la présente invention n'est pas spécifiquement limité, et il est arbitrairement choisi parmi les procédés tels qu'un procédé d'addition d'une huile de silicone à de l'eau contenant les polysaccharides et un tensioactif et des composants hydrophiles tout en les agitant vigoureusement, un procédé d'agitation vigoureuse d'eau contenant des polysaccharides, un tensioactif et une huile de silicone, et un procédé d'addition d'un tensioactif à une huile de silicone, et d'addition d'une solution aqueuse de polysaccharides à l'huile de silicone sous agitation. Dans un procédé représentatif de production de la composition cosmétique mélangée avec l'huile de silicone, une solution aqueuse homogène est préparée par addition de polysaccharides et d'un tensioactif à de l'eau, la solution aqueuse homogène est chauffée à une température de 40 à 70°C, et une huile de silicone y est ajoutée sous une agitation à environ 5 000 tours par minute avec un homomélangeur pour former une émulsion. Le diamètre de particule de l'émulsion est mesuré avec un dispositif de mesure de la distribution de taille de particule par diffraction au laser LP-500 (Horiba Co.), ou analogue, sous agitation. L'émulsion est agitée en continu jusqu'à ce que le diamètre moyen de particule soit dans une gamme de 1 à 50 µm, de préférence de 2 à 20 µm. La température peut être ajustée à une température qui facilite l'agitation et le mélange selon la viscosité de l'huile de silicone à utiliser, et les types des polysaccharides et du tensioactif, et elle est habituellement dans la gamme de 20 à 70°C. La durée d'agitation est choisie de façon à donner le diamètre de particule recherché, et elle est habituellement dans la gamme de 5 minutes à 2 heures.

De plus, la composition cosmétique mélangée avec l'huile de silicone de la présente invention peut être une composition cosmétique en émulsion composite de type E/H/E ou H/E/H mélangée avec l'huile de silicone, ou une composition cosmétique de type microcapcule mélangée avec l'huile de silicone. La composition cosmétique mélangée avec une huile de silicone de la présente invention peut être une nano-émulsion ou une nano-suspension ayant des gouttes d'eau, des gouttes d'huile ou des pigments dont les tailles sont inférieures à 1 µm.

Par exemple, on peut obtenir une composition cosmétique en émulsion composite E/H/E mélangée avec une huile de silicone en formant un gel à partir d'un composant formant un gel tel que des alcools polyfonctionnels et d'un tensioactif lipophile ; en formant une émulsion E/H à partir du gel, d'un composant hydrophile contenant un composant d'âme hydrophile et d'un composant lipophile, et en dispersant et en émulsifiant l'émulsion E/H dans une phase aqueuse externe contenant un composant filmogène hydrophile.

Et, on peut obtenir une composition cosmétique en émulsion composite H/O/H ou de type microcapsule E/H/E mélangée avec une huile de silicone en préparant une émulsion H/E ou O/E à partir d'une phase huileuse interne et d'une phase aqueuse dans laquelle on a dissous au préalable un agent gélifiant polymère hydrophile par chauffage, à une température non inférieure à la température de solidification de l'agent gélifiant, et en préparant l'émulsion H/E/H dans laquelle l'émulsion H/E est dispersée et émulsifiée dans une phase huileuse externe à une température non inférieure à la température de solidification de l'agent gélifiant, ou en préparant l'émulsion E/H/E dans laquelle l'émulsion E/H est dispersée et émulsifiée dans une phase aqueuse externe à une température non inférieure à la température de solidification de l'agent gélifiant.

Les appareils utilisés pour la dissolution, ou l'émulsification et la dispersion de l'huile de silicone ou du composant lipophile de la composition cosmétique mélangée avec une huile de silicone de la présente invention, englobent un agitateur à ailette, un dispositif de dissolution, un homomélangeur, un broyeur à billes, un broyeur à sable, un dispositif de dispersion ultrasonique, une pétrisseuse, un mélangeur en ligne, un homogénéisateur vertical, un microfluidificateur, une homogénéisateur haute pression et un dispositif de nano-atomisation, et ils sont convenablement choisis pour l'utilisation.

Dans la composition cosmétique mélangée avec une huile de silicone de la présente invention, de l'eau pure, de l'eau de source chaude, de l'eau de mer profonde, des solvants organiques, des agents huileux, des agents épaississants, des agents colorants, des agents de conservation de l'humidité, des astringents, des agents de blanchiment, des agents anti-UV, des agents anti-inflammatoires, des agents activant les cellules de la peau, des agents carbonatés, des agents de prévention de l'oxydation, des agents antiseptiques et des bactéricides, des agents chélatants, des agents de prévention de la décoloration, des agents tampon et des parfums, peuvent être utilisés dans une gamme qui n'altère pas l'effet de l'invention, selon l'application de la composition cosmétique.

Des exemples des solvants organiques qui peuvent être utilisés dans la composition cosmétique de la présente invention englobent l'éthanol, l'acétone, l'acétate d'éthyle, l'acétate de butyle, le 1,3-butylèneglycol, l'éthylèneglycol, le diéthylèneglycol, le triéthylèneglycol, le polyéthylèneglycol, le propylèneglycol, le dipropylèneglycol, la glycérine, le butanol, le propanol, etc.

Des exemples des agents huileux englobent les hydrocarbures, les cires, les acides gras, les alcools supérieurs, les esters d'acides gras, les esters d'acides organiques, les glycérides, les hydrocarbures fluorés, etc. Plus spécifiquement, des exemples d'hydrocarbures englobent le squalane, le squalène, la cérésine, la paraffine, la cire de paraffine, la paraffine liquide, le pristane, le polyisobutyène, la cire microcristalline, la vaseline, etc. Des exemples de cires englobent la cire d'abeille, la cire de carnauba, la cire de candélilla, la cire de requin, etc., des exemples d'huiles animales englobent la suif de boeuf, la graisse de jambe de bétail, la graisse d'os de bétail, la suif de boeuf durcie, une huile hydrogénée, l'huile de tortue de mer, le lard, la graisse de cheval, l'huile de vison, l'huile de foie, l'huile de jaune d'oeuf, etc. des exemples de dérivés de lanoline englobent la lanoline, la lanoline liquide, la lanoline réduite, l'alcool de lanoline, la lanoline dure, l'acétate de lanoline, l'acide gras isopropylique de lanoline, l'alcooléther de lanoline et de POE, l'acétate d'alcool de lanoline et de POE, l'acide gras de lanoline de polyéthylèneglycol, l'alcooléther de lanoline de POE hydrogéné, etc. des exemples d'acides gras englobent l'acide laurique, l'acide myristique, l'acide palmitique, l'acide stéarique, l'acide béhénique, l'acide undécylénique, l'acide oléique, l'acide arachidonique, l'acide docosahexanoique (DHA), l'acide isostéarique, lacide 1,2-hydroxystéarique, etc., des exemples d'alcools supérieurs englobent l'alcool laurylique, l'alcool myristylique, l'alcool palmitylique, l'alcool stéarylique, l'alcool béhénylique, l'alcool hexadécylique, l'alcool oléylique, l'alcool isostéarylique, l'hexyldodécanol, l'octyldodécanol, l'alcool cétostéarylique, le 2-décyl-tétradécinol, le cholestérol, le phytostérol, le sitostérol, le lanostérol, le cholestéroléther de POE, le monostéarylglycérineéther (l'alcool batylique), etc., des exemples d'esters d'acides gras englobent l'adipate de diisobutyle, l'adipate de 2-hexyldécyle, l'adipate de 2-heptylundécyle, le monoisostéarate de N-alkylglycol, l'isostéarate d'isocétyle, le triisostéarate de triméthylolpropane, le di-2-éthylhexanoate d'éthylèneglycol, le 2-éthylhexanoate de cétyle, le tri-2-éthylhexanoate de triméthylolpropane, le tétra-2-éthylhexanoate de pentaérythritol, l'éthylhexanoate de cétyle, l'octyldodécylester de gomme, l'oléate d'oléyle, l'oléate de dodécyloctyle, l'oléate de décyle, le dicaprate de néopentylglycol, le citrate de triéthyle, le succinate de 2-éthylhexyle, l'acétate d'amyle, l'acétate d'éthyle, l'acétate de butyle, le stéarate d'isocétyle, le stéarate de butyle, le sébacate de diisopropyle, le sébacate de di-2-éthylhexyle, le lactate de cétyle, le lactate de myristyle, le palmitate d'isopropyle, le palmitate de 2-éthylhexyle, le palmitate de 2-hexyldécyle, le palmitate de 2-heptylundécyle, le 12-hydroxystéarate de cholestéryle, l'ester d'acide gras de dipentaérythritol, le myristate d'isopropyle, le myristate d'octyldodécyle, le myristate de 2-hexyldécyle, le myristate de myristyle, le diméthyloctanate d'hexyldécyle, le laurate d'éthyle, le laurate d'hexyle, etc., des exemples d'ester d'acide aminé englobent le 2-octyldodécylester d'acide N-lauroyl-L-glutamique, des exemples d'ester d'acide organique englobent le malate de di-isostéaryle, etc., des exemples de glycérides englobent l'acétylglycéride, le tri-isooctanoate de glycéride, le tri-isostéarate de glycéride, le tri-isopalmitate de glycéride, le tri-2-éthylhexanoate de glycéride, le monostéarate de glycéride, le di-2-heptylundécanoate de glycéride, le trimyristate de glycéride, etc. et des exemples d'hydrocarbure fluoré englobent le polyperfluoroéther, la perfluorodécaline, le perfluorooctane, etc.

Des exemples des agents épaississants englobent les polymères supérieurs naturels solubles dans l'eau et gonflables dans l'eau, les polymères supérieurs semi-synthétiques, les polymères supérieurs synthétiques et les composés minéraux. Plus spécifiquement, des exemples de polymères supérieurs naturels englobent la gomme arabique, la gomme guar, la gomme de karaya, la mousse perlée, la pectine, le fucoidan, la gomme de graine de coine, la gomme adragante, la gomme de graine de caroube, le galactomannan, la gomme xanthane, le curdlan, la gomme gélifiante, le gel fucacé, la caséine, la gélatine, l'amidon, le collagène, etc., des exemples de polymères supérieurs semi-synthétiques englobent la méthylcellulose, l'éthylcellulose, la méthylhydroxypropylcellulose, la carboxyméthylcellulose, l'hydroxyméthylcellulose, l'hydroxypropylcellulose, la carboxyméthylcellulose de sodium, l'alginate de propylèneglycol, etc., des exemples des polymères synthétiques englobent le poly(alcool vinylique), la polyvinylpyrrolidone, un polymère carboxyvinylique, le poly(acide acrylique), le poly(acrylate de sodium), le poly(acrylate de potassium), le poly(oxyde d'éthylène), un copolymère oxyde d'éthylène-oxyde de propylène, etc., et des exemples de composés minéraux englobent la smectite, la montmorillonite, la bentonite, la laponite, l'hetorite, etc., Un, deux ou plusieurs de ces substances peuvent être utilisées.

Des exemples de l'agent colorant englobent les colorants synthétiques organiques, les colorants naturels, les pigments, etc. Plus spécifiquement, des exemples des colorants synthétiques organiques englobent yellow N° 5 et red N° 505, etc., des colorants azoïques, red N° 213 et red N° 230, etc., des colorants xanthène, yellow N° 204, etc., des colorants quinoléine, blue N° 1, etc., des colorants triphényméthane, green N° 201, etc., des colorants anthraquinone, le colorant indigo, etc., et des exemples de colorants naturels englobent les carotènes, la carthamine, les cochinéales, etc. Des exemples de pigments englobent un pigment de verni, un pigment organique, un pigment de couleur, un pigment blanc, un pigment diluant, un pigment nacré, un pigment à lustre métallique, un pigment de paillettes de verre, un pigment minéral enrobé de métal, un pigment de résine, des poudres de polymères supérieurs, un pigment fonctionnel, etc. Des exemples du pigment de verni englobent red N° 202, red N° 204, red N° 206, red N° 207, red N° 208, red N° 220, yellow N° 5, red N° 230, etc., des exemples de pigments organiques englobent red N° 228 de pigment azoïque, red N° 226 de pigment indigo, blue N° 404 de pigment phtalocyanine, etc., des exemples de pigment de couleur englobent les oxydes de fer ayant différentes couleurs, tels que l'oxyde de fer rouge, l'oxyde de fer jaune, l'oxyde de fer noir, le bleu outre-manche, le bleu de milori, l'oxyde de chrome, l'hydroxyde de chrome, l'oxyde de magnésium, l'oxyde de cobalt, l'oxyde de cobalt-titane, le noir de carbone, le violet de manganèse, le violet de cobalt, etc., des exemples de pigment blanc englobent le dioxyde de titane, l'oxyde de zinc, etc., des exemples de pigment diluant englobent le pigment à base de mica qui est utilisé pour conserver des configurations, la capacité de dilution et l'adhérence de produits, et pour ajuster le lustre, la teinte de couleur ou analogue de produits (diluant), etc. Des exemples de pigment à base de mica englobent le mica, la muscovite, le mica synthétique, la phlogopite, le mica rouge, la biotite, le mica de lithium, la smectite, la séricite, le talc, le kaolin, la montmorillonite, la bentonite, une zéolite, le carbonate de magnésium, le carbonate de calcium, l'acide silicique, l'acide silicique anhydre, le silicate d'aluminium, le silicate de magnésium, le silicate de magnésium-aluminium, le silicate d'aluminium contenant du soufre, le silicate de calcium, le silicate de baryum, le silicate de strontium, l'oxyde d'aluminium, le sulfate de baryum, etc., des exemples de pigment nacré englobent le dioxyde de titane recouvrant du mica, les paillettes d'écailles de poisson, l'oxychlorure de bismuth, un pigment recouvert d'oxyde de fer au lieu du dioxyde de titane, le pigment portant une couche de couverture de titanate recouverte d'un autre pigment d'une couleur transparente différente, etc., qui sont utilisés pour donner un lustre perlé, les couleurs de l'arc-en-ciel et une sensation métallique à des produits. Des exemples de pigment à lustre métallique englobent un pigment de poudre métallique colorée préparé par coloration d'une poudre d'aluminium, d'une poudre de laiton, d'une poudre de cuivre, d'une poudre d'étain, d'une poussière d'or, d'une poudre d'argent et d'une autre poudre métallique, etc., et un pigment à paillettes de verre n'est pas spécifiquement limité, à condition que le métal soit recouvert de verre en paillette. Le pigment minéral enrobé de métal est un pigment minéral qui est recouvert d'un métal et/ou d'un oxyde métallique par évaporation métallique, et des exemples de celui-ci englobent l'oxyde de fer recouvrant de l'aluminium, de l'oxyde de fer recouvrant du mica, de l'aluminium-manganèse recouvrant de l'oxyde de fer à base de mica, etc. Un pigment de résine est constitué d'une paillette de résine préparée par coloration d'un film de résine et par découpage du film de résine coloré en paillettes, des exemples de pigment de résine englobent une poudre de film de polyester, un film stratifié poly(téréphalate d'éthylène)-aluminium-époxy, un film stratifié poly(téréphalate d'éthylène)-polyoléfine, le poly(méthacrylate de méthyle), un stratifié poly(téréphalate d'éthylène)-poly(méthacrylate de méthyle), une poudre de nylon, etc., et des exemples de pigment fonctionnel englobent le nitrure de bore, la phogopite de bore synthétique, un pigment photochromique, une poudre composite à grain fin, etc. La forme de ces pigments n'est pas spécifiquement limitée, et elle peut être choisie arbitrairement parmi une forme granulaire, une forme de feuille, une forme de tige, etc., selon le but et le type de pigment à utiliser.

La taille du pigment n'est pas spécifiquement limitée, et elle peut être arbitrairement choisie selon le but et le type de pigment à utiliser. Habituellement, dans le cas de la forme granulaire, un pigment ayant un diamètre moyen de particule s'échelonnant de 0,01 µm à 5 000 µm est utilisé et, dans le cas d'une forme en paillette ou une forme en tige, un pigment ayant un diamètre de particule plus long s'échelonnant de 0,1 à 5 000 µm est utilisé.

Des exemples d'humidifiant englobent un polyol, tel que la glycérine, le propylèneglycol, le dipropylèneglycol, le 1,3-butylèneglycol, le polyéthylèneglycol, le sorbitol, etc., le carbonate de pyrrolidone, le lactate, etc., en tant qu'ingrédients principaux de NMF (facteur humidifiant naturel), l'hyaluronate, le sulfate de chondroitine, l'héparine, etc., en tant que mucopolysaccharides, l'urée, la cystéine, la sérine, etc., en tant qu'acides aminés, divers types d'extraits végétaux, etc.

Des exemples d'astringents englobent le phénolsulfonate de zinc, le phoslosulfonate de sodium, les extraits végétaux tels que l'arnica, l'aubébine, la cinchonille, la sauge, la tilia miqueliana, le ginseng, le junipérus communis, le romarin, l'hypericum, l'arbre aux quarante écus, le mélise, l'ononis spinosa, le marronnier, le swertia japonica, l'ail, la camomille, le thym, la menthe, l'ortie, le poivre rouge, le gingembre, le houblon, le marron d'Inde, la Lavandula vera, la carotte, la moutarde en feuille, le cinnamomum casia, le pin, le cnidium officinale, le sureau, l'ostericum sieboldii, le scopolia, le bouton, la myrica, l'houttuynia, l'os d'orysa sativa, la plaquemine amère, le calendula officinalis, le coquelicot, le gertiana scabra, le raisin, la glehnia, l'orange, le citron, l'iris, l'orange d'été du Japon, l'hamamélis, le trèfle doux, l'anis, le poivre du Japon, la pivoine, l'eucalyptus, l'artemisia vulgaris indica, l'isodon japonicus, le rie, la sophora angustifolia, le pain, le clou de girofle, la feuille de châtaigne, la racinde de scutellaria, la sauge, les racines tubéreuse de polygonom multiflorum, le coptidis rhizoma, le phellodendri cortex, le scuttellaria baicalensis GEORGI, l'Houttuyniae herba, l'aurantii nobilis pericarpium, la carotte, la pivoine, le codonopsitis radix, le propolis, l'alisma rhizome, le tannin, le goudron de bois de bouleau, le gelée royale, l'extrait de levure, etc. L'une de ces substances peut être utilisée, ou deux ou plusieurs de celles-ci peuvent être utilisées en combinaison. Habituellement, la quantité des astringents s'échelonne de 0,001 à 5 % en poids, de préférence de 0,01 à 3 % en poids, par rapport à la quantité totale de la composition cosmétique.

Des exemples des agents de blanchiment englobent un inhibiteur de tyrosinase, un antagoniste de l'endothéline, un inhibiteur d'α-MSH, la glabridine, le glablène, la liquilitine, l'isoliquilotine, l'acide ellagique, les sels de l'acide ellagique, les dérivés de l'acide ellagique, l'acide kojique, les sels de l'acide kojique, les dérivés de l'acide kojique, l'hydroquinone telle que l'arbutine, les sels d'hydroquinone, les dérivés d'hydroquinone, la cystéine, les sels de cystéine, les dérivés de cystéine, la vitamine C telle que l'acide ascorbique, l'ascorbate de sodium, le stéarate d'ascorbyle, le palmitate d'ascorbyle, le dipalmitate d'ascorbyle, l'ascorbyl-2-phospahte de magnésium, les sels de vitamine C, les dérivés de vitamine C, la glutathione, les sels de glutathione, les dérivés de glutathione, la résorcine, les sels de résorcine, les dérivés de résorcine, le rucinol, la néoagarobiose, l'oligosaccharide d'agarose, et les extraits végétaux tels que l'extrait d'asperge, l'extrait d'althaea officinalis, l'extrait de bistorta major, l'extrait d'artemisiae capillaris flos, l'extrait de pois, l'extrait de fruit de rose de chien, l'extrait de racine de scutellaria, l'extrait d'ononis spinosa, l'extrait d'algues, l'extrait de firethorm, l'extrait de réglisse, l'extrait de mûre, l'extrait de racine de sophora, l'extrait de sucre brun, l'extrait de millettja triculate, l'extrait d'acanthopanacis cortex, l'extrait de germe de mais, l'extrait d'asiasari radix, l'extrait d'aubépine, l'extrait de nomame herba, l'extrait de pivoine, l'extrait de lilas, l'extrait de fleur d'inule, l'extrait d'écorce de mûrier, l'extrait de soja, l'extrait de placenta, l'extrait d'aralia elata, l'extrait de thé, l'extrait d'angélica acutiloba, l'extrait de mélase, l'extrait de rosa polyantha, l'extrait d'ampelopsis japonica, l'extrait de grain de raisin, l'extrait de bouleau, l'extrait de fleur de manite, l'extrait de houblon, l'extrait de rosa rugosae flos, l'extrait de chaenomelis fructus, l'extrait de saxifraga, l'extrait de coicis semen, l'extrait de rakanta, etc. une de ces substances peut être utilisées, ou deux ou plusieurs d'entre elles peuvent être arbitrairement utilisées. Habituellement, la teneur des agents de blanchiment s'échelonne de 0,01 à 10 %. Lorsque les extraits végétaux ou analogue sont utilisés sous forme d'un liquide extrait, la teneur ci-dessus est la quantité convertie en extrait solide séché.

Les agents anti-ultraviolets englobent les absorbants d'ultraviolets et les agents dispersant les rayons ultraviolets. Des exemples d'absorbant d'ultraviolets englobent les absorbants d'ultraviolets à base d'acide para-aminobenzoïque, les absorbants d'ultraviolets à base d'acide cinnamique, les absorbants d'ultraviolets à base d'acide salicylique, les absorbants d'ultraviolets à base de benzophénone. Des exemples des absorbants d'ultraviolets à base d'acide para-aminobenzoïque englobent l'acide para-aminobenzoïque, le para-aminobenzoate de glycéryle, le para-aminobenzoate d'éthyldihydropropyle, le para-aminobenzoate de para-diméthyle, le para-aminobenzoate d'octyl-para-méthyl, le para-aminobenzoate d'éthyle, le para-aminobenzoate d'isobutyle, etc., des exemples des absorbants d'ultraviolets à base d'acide cinnamique englobent le para-méthoxycinnamate d'isopropyle, le para-cinnamate de diisopropyle, le méthoxycinnamate d'octyle, le cinnamate de di-para-méthoxy, le mono-2-éthylhexanoate de glycéryle, etc., des exemples des absorbants d'ultraviolets à base d'acide salicylique englobent le salicylate d'homomenthyle, le salicylate d'octyle, le salicylate de phényle, la triéthanolamine d'acide salicylique, le salicylate d'amyle, le salicylate de benzile, le salicylate de p-tert-butylphényle, le salicylate d'éthylèneglycol, l'acide salicylique, etc., des exemples des absorbants d'ultraviolets à base de benzophénone englobent la dihydroxybenzophénone, la tétrahydroxybenzophénone, l'oxybenzone, l'acide oxybenzonesulfonique, l'hydroxyméthoxybenzophénone-sulfonate de sodium, la dihydroxydiméthoxybenzophénone, la 2-hydroxychlorobenzophénone, la dioxybenzophénone, le dihydroxydiméthoxybenzophénonedisulfonate de sodium, la 2-hydroxy-4-méthoxy-4'-méthylbenzophénone l'octabenzone, l'acide urocanique, l'urocanate d'éthyle, le 4-tert-4'-méthoxydibenzoylméthane, le 2-(2'-hydroxy-5'-méthylphényl)benzotriazole, l'acide anthranilique, etc.

Des exemples d'agents anti-inflammatoires englobent l'oxyde de zinc, le soufre, les dérivés du soufre, l'acide glycyrrhizinique, les dérivés et les sels de l'acide glycyrrhizinique, tels que le glycyrrhigate dipotassique et le glycyrrhigate de monoammonium, l'acide glycyrrhétique, les dérivés et les sels de l'acide glycyrrhétique tels que l'acide β-glycyrrhétique, le glycyrrhétinate de stéaryle, le 3-succinyloxyglycyrrhétinate disodique, etc., l'acide tranexamique, le sulfate de chondroïtine, l'acide méfénamique, la phénylbutazone, l'indométacine, l'ibuptofen, le cétoprofen, l'allantoïne, le quaiazulène et les dérivés et les sels de celui-ci, divers types de microbes, les extraits animaux, les extraits végétaux, etc.

Des exemples d'activateur de la peau (cellule) englobent l'acide désoxyribonucléique, les sels de l'acide désoxyribonucléique, les dérivés d'acide adénylique, tels que l'adénosinetriphosphate, l'adénosinephosphate, les sels de celui-ci, l'acide ribonucléique, les sels de celui-ci, l'AMP cyclique, le GMP cyclique, la flavine, l'adéninedinucléotide, la quanine, l'adénine, la cytosine, la thymine, la xanthine et les dérivés de celle-ci, la caféine, la théophylline et les sels de celle-ci, le rétinol, les dérivés de rétinol tels que le palmitate de rétinol, l'acétate de rétinol, etc., le rétinal, les dérivés de rétinal tels que le déshydrorétinal, etc., la carotène, la vitamine A telle que le caroténoïde, etc., la thiamine, les sels de thiamine tels que le chlorhydrate de thiamine, le sulfate de thiamine, etc., la riboflavine, les sels de riboflavine tels que l'acétate de riboflavine, etc., la pyridoxine, les sels de pyridoxine tels que le chlorhydrate de pyridoxine, le dioctanoate de pyridoxine, etc., le flavineadéninedinucléotide, la cyanocobalamine, les acides foliques, l'acide nicotinique, les dérivés de l'acide nicotinique tels que le nicotinamide, l'acide benzilnitotinique, etc., la vitamine B telle que la choline, etc., l'acide γ-linolénique et les dérivés de celui-ci, l'acide éicosapentaénoïque et les dérivés de celui-ci, l'estradiol et les dérivés et les sels de celui-ci, les acides organiques tels que l'acide glycolique, l'acide succinique, l'acide lactique, l'acide salicylique, etc., et les dérivés et les sels de ceux-ci.

Des exemples d'agents antibactériens englobent l'acide benzoïque, le benzoate de sodium, l'acide carbolique, l'acide sorbique, le sorbate de potassium, l'ester d'acide para-oxybenzoïque, le para-chlorométacrésol, l'hexachlorophène, le chlorure de benzalkonium, le chlorure de chlorhexidine, le trichlorocarbanilide, le guaternum, le bis(2-pyridylthio-1-oxyde) de zinc, le phénoxyéthanol, le thianthol, l'isopropylméthylphénol, etc.

Des exemples d'antioxydants englobent la vitamine A tels que le rétinol, le déshydrorétinol, l'acétate de rétinyle, le palmitate de rétinyle, le rétinal, l'acide rétinoïque, une huile de vitamine A, les dérivés et les sels de celle-ci, les caroténoides tels que l'α-carotène, la β-carotène, la γ-carotène, la cryptoxanthine, l'astaxanthine, la fucoxanthine, etc., et les dérivés et les sels de ceux-ci, la vitamine B telle que la pyridoxine, le pyridoxal, l'ester pyridoxal-5-phosphate, la pyridoxamine, etc. et les dérivés et les sels de celle-ci, la vitamine C telle que l'acide ascorbique, l'ascorbate de sodium, le stéarate d'ascorbyle, le palmitate d'ascorbyle, le dipalmitate d'ascorbyle, le phosphate d'acide ascorbique de magnésium, etc., et les dérivés et les sels de celle-ci, la vitamine D telle que l'ergocalciférol, le cholecalciférol, le 1,2,5-dihydroxy-cholecalciférol, etc., les dérivés et les sels de celle-ci, la vitamine E telle que l'α-tocophérol, le β-tocophérol, le γ-tocophérol, le δ-tocophérol, l'α-tocotriénol, le β-tocotriénol, le γ-tocotriénol, le δ-tocotriénol, l'acétate de tocophérol, le nicotinate de tocophérol, etc., les dérivés et les sels de celle-ci, le tororoks (dérivés de la vitamine E solubles dans l'eau), les dérivés et les sels de ceux-ci, le dihydroxytoluène, le butylhydroxytoluène, le butylhydroxyanisol, le dibutylhydroxytoluène, l'acide α-lipoïque, l'acide déshydralipoïque, la glutathione, les dérivés et les sels de celle-ci, l'acide urique, l'acide érythorbique, les dérivés et les sels de celui-ci tels que l'érythorbate de sodium, etc., l'acide gallique, les dérivés et les sels de celui-ci tels que le gallate de propyle, etc., la rutine, les dérivés et les sels de celle-ci tels que l'α-glycosylrutine, etc., le tryptophane, les dérivés et les sels de celui-ci, l'histidine, les dérivés et les sels de celle-ci, les dérivés et les sels de cystéine tels que la N-acétylcystéine, la N-acétylhomocystéine, la N-octanoylcystéine, l'ester méthylique de N-acétylcystéine, etc., les dérivés et les sels de cystine tels que l'ester diméthylique de N,N'-diacétylcystine, l'ester diméthylique de N,N'-dioctanoylcystine, l'ester diméthylique de N,N'-dioctanoylhomocystine, etc., la carnosine, les dérivés et les sels de celle-ci, l'homocarnosine, les dérivés et les sels de celle-ci, l'ansérine, les dérivés et les sels de celle-ci, la calcinine, les dérivés et les sels de celle-ci, les dérivés et les sels de dipeptide ou de tripeptide contenant de l'histidine et/ou du triptophan et/ou de l'histamine, les flavonoides tels que la flavanone, la flavone, l'anthocyanine, l'anthocyanidine, le flavonol, le quelcitron, la myricétine, la phycétine, l'hamamelis tannin, la catéchine, l'épicatéchine, la gallocatéchine, l'épigallocatéchine, le gallate d'épicatéchine, le gallate d'épigallocatéchine, etc., l'acide tannique, l'acide cafféinique, l'acide férulique, l'acide protocatécuïque, la chalcone, l'oryzanol, le carnot sole, le sesamole, la sésamine, la sésamoléine, la zingérone, la curcumine, la tétrahydrocurcumine, le chlobamide, le désoxychlobamide, le shoga-ol, la capsaicine, le vanillylamide, l'acide ellagique, le bromophénol, la flavoglassine, la mélanoidine, la riboflavine, l'ester butylique de riboflavine, le flavinemononucléotide, le flavineadéninenucléotide, l'ubiquinone, l'ubiquinol, le mannitol, la bilirubine, le cholestérol, l'ébsélène, le sélénométhionine, la cérulplasmine, la transferrine, la lactoferrine, l'albumine, la bilirubine, la superoxydedismutase, la catalase, la glutathioneperoxydase, la métallothionéine, l'O-phosphono-pyridoxylidène-rhodamine, l'acide N-(2-hydroxybenzyl)aminé, les dérivés et les sels de celui-ci, l'acide N-(4-pyridoxylméthylène)aminé, les dérivés et les sels de celui-ci, qui sont décrits dans le brevet américain N° 5 594 012, etc. La teneur en antioxydants dépend des types de ceux-ci de façon à ne pas les déterminer de façon égale. Mais, habituellement, la teneur de ceux-ci s'échelonne de 0,01 à 10 %. Lorsque l'extrait végétal ou analogue est utilisé comme liquide extrait, la teneur décrite ci-dessus est exprimée en quantité convertie d'un extrait solide sec.

Des exemples de parfums englobent les parfums naturels et les parfums synthétiques. Des exemples de parfums naturels englobent les parfums végétaux naturels tels que l'huile de rose, l'huile de jasmin, l'huile de néroli, l'huile de lavande, l'huile de tuberose, l'huile de Ylang Ylang, l'huile de sauge sclarée, l'huile de clou de girofle, l'huile de menthe pouvrée, l'huile de géranium, l'huile de patchouli, l'huile de santal, l'huile de cannelle, l'huile de coriandre, l'huile de muscade, l'huile de pin, l'huile de vanille, l'huile de balsam Peru, l'huile de banane, l'huile de pomme, l'huile de fenouil, l'huile de graine de tonca, l'huile de poivre, l'huile de citron, l'huile d'orange, l'huile de bergamote, l'huile d'opopanax, l'huile de vétiver, l'huile d'iris, l'huile de chêne, l'huile d'anise, l'huile de bois de rose, etc., et les parfums animaux naturels tels que l'huile de vison, l'huile de civet, l'huile de ricin, l'huile d'ambre gris, etc. Des exemples de parfums synthétiques englobent la limonène, le β-caryophylène, le cis-3-hexénol, le linalool, le farnésol, l'alcool β-phényléthylique, le géraniol, le citronellol, le terpinéol, le menthol, le santalol, le bacdanol, le puramanol, le lyranol, le lilial, le 2,6-nonadiénal, le citral, l'aldéhyde d'α-hexylcinnamique, la β-ionone, la 1-carvone, la cyclopentadécanone, la damascone, la méthylionone, l'irone, l'IOS E-super, l'acétylcédrène, la muscone, l'acétate de benzyle, le dihydrojasmonate de méthyle, le jasmonate de méthyle, l'acétate de linalyle, le benzoate de benzyle, la γ-undécalactone, la jasminelactone, le cyclopentadécanolide, le brassylate d'éthylène, le galactsolide, l'ambroxane, l'oxyde de rose, l'eugénol, l'indole, le phénylacétaldéhyediméthylcétal, l'aurantiol (base de schiff), etc., et plusieurs de ces parfums sont utilisés en combinaison selon les buts de ceux-ci.

### [Exemples]

Dans la suite, la présente invention sera expliquée en détail en référence à plusieurs modes de réalisation, mais la présente invention n'est pas limitée à ces modes de réalisation.

### [Polysaccharide utilisé dans les expériences]

### (A-1 : Polysaccharide d'un produit d'un micro-organisme de la souche Alcaligenes latus B-16 (produit brut))

On dissout dans une eau à ions échangés, 40,0 g de glucose [Wako Pure Chemical Industries, Ltd., regent], 4,0 g d'hydrogénophosphate dipotassique [Wako Pure Chemical Industries, Ltd., regent], 2,0 g de dihydrogénophosphate de potassium [Wako Pure Chemical Industries, Ltd., regent], 0,1 g de chlorure de sodium [Wako Pure Chemical Industries, Ltd., regent], 0,2 g de sulfate de magnésium [Wako Pure Chemical Industries, Ltd., regent], 1,0 g de nitrate de potassium [Wako Pure Chemical Industries, Ltd., regent] et 1,5 g d'extrait de levure [OXOID Co., Ltd.], et on ajuste la solution aqueuse obtenue à un pH de 6,5 en utilisant de l'hydroxyde de sodium ou de l'acide sulfurique, de sorte que le volume total soit de 1 litre. On transfère 150 ml de la solution aqueuse obtenue dans un ballon conique de 500 ml et on la stérilise par passage à l'autoclave à 121°C pendant 15 minutes. Ensuite, on abaisse la température de la solution à la température ambiante, et on inocule la souche Alcaligenes latus B-16 (FERM BP-2015) dans la solution dans le ballon. Et, on soumet la solution à une culture sous agitation à 30°C pendant 6 jours (180 tours par minute). Après la culture, on y ajoute environ trois volumes d'alcool isopropylique et on les agite pour les mélanger. On filtre l'agglomérat précipité résultant, on le récupère et on le sèche sous pression réduite pour obtenir des polysaccharides d'un produit d'un micro-organisme de la souche Alcaligenes latus B-16 (A-1). Les polysaccharides obtenus comprennent un polysaccharide constitué de fucose, de glucose, d'acide glucuronique et de rhamnose dans un rapport molaire de 1:2:1:1, en tant que composant principal, et un autre polysaccharide constitué de fucose et de mannose dans un rapport molaire de 1:2. Le rapport du premier polysaccharide au second polysaccharide est de 7:1 (rapport en poids). On hydrolyse les polysaccharides avec de l'acide sulfurique, et on analyse les monosaccharides résultants avec une chromatographie liquide haute pression (HPLC).

### (A-2 : produit purifié de A-1 ci-dessus)

On prépare une solution aqueuse de 0,5 % en poids des polysaccharides A-1, et on y ajoute une solution aqueuse d'hydroxyde de sodium jusqu'à un pH de 12. On traite la solution aqueuse obtenue en utilisant des colonnes de résine à ions échangés "DIAON HPA-75 (OH-) (marque de commerce)" (fabriquée par Nippon Rensui Co.) à 8 Ru ou moins, et on la filtre avec un auxiliaire de filtration "Radiolight RL700" et un filtre à membrane de 5 µm pour éliminer les protéines, les acides nucléiques et les microbes. Après avoir ajusté le liquide filtré à pH 7 avec de l'acide chlorhydrique dilué, on réduit la pression du liquide, et on concentre le liquide. Ensuite, on précipite les polysaccharides en utilisant de l'acétone, et on les lave avec dix volumes d'acétone, pour obtenir les polysaccharides (A-2) constitué de fucose, de glucose, d'acide glucuronique et de rhamnose dans un rapport molaire de 1:2:1:1, et ayant une masse moléculaire élevée de cinquante million.

### [Huile de silicone]

B-1 : diméthylpolysiloxane (viscosité de 50 mPa.s, 25°C, fabriqué par Shin-Etsu Chemical Co., Ltd.)
B-2 : diméthylpolysiloxane (viscosité de 100 mPa.s, 25°C, fabriqué par Shin-Etsu Chemical Co., Ltd.)
B-3 : diméthylpolysiloxane (viscosité de 50 000 mPa.s, 25°C, fabriqué par Shin-Etsu Chemical Co., Ltd.)
B-4 : méthylphénylpolysiloxane (viscosité de 500 mPa.s, 25°C, fabriqué par Nihonunica Corporation)
B-5 : copolymère diméthylpolysiloxane-méthylsiloxane polyéthoxylé (viscosité de 1 600 cSt, "SH 3775C (marque de commerce)" fabriqué par Toray Dow Corning Silicone Co., Ltd.)

### [Préparation de la lotion 1] (ne faisant pas partie de la présente invention)

| (N°) | (Composants de mélange) | (% en poids) |
|---|---|---|
| 1. | éthanol | 5,00 |
| 2. | glycérine | 3,00 |
| 3. | 1,3-butylèneglycol | 3,00 |
| 4. | glycyrrhizine dipotassique | 0,20 |
| 5. | monolaurate de saccharose | |
| (Cosmelike L-160, fabriqué par Dai-ichi Kogyo Seiyaku Co., Ltd.) | | 0,50 |
| 6. | polysaccharide (A-1) | 0,02 |
| 7. | méthylphénylpolysiloxane (B-4) | 2,00 |
| 8. | para-aminobenzoate de méthyle | 0,10 |
| 9. | eau pure | complément |

On prépare un mélange 1 en agitant et en mélangeant les composants de mélange N° 1 à 3, 5 et 7. De même, on prépare un mélange 2 en agitant et en mélangeant les composants de mélange N° 4, 6, 8 et 9. On ajoute le mélange 1 au mélange 2 tout en agitant le mélange 2 à 5 000 tours par minute avec un homomélangeur pour préparer une émulsion. On obtient ainsi une lotion 1 contenant 1,0 % de polysaccharide (A-1) par rapport à l'huile de silicone.

### [Préparation de la lotion 2]

On obtient la lotion 2 par le même procédé que pour la lotion 1, sauf que l'on remplace 0,02 % de polysaccharide (A-1) en tant que composant de mélange N° 6 dans la lotion 1 par 0,02 % de polyacrylate.

### [Préparation de la lotion 3] (ne faisant pas partie de présente invention)

| (N°) | (Composants de mélange) | (% en poids) |
|---|---|---|
| 1. | éthanol | 14,00 |
| 2. | glycérine | 4,00 |
| 3. | 1,3-butylèneglycol | 2,00 |
| 4. | oxyde de fer | 0,05 |
| 5. | oxyde de zinc | 0,50 |
| 6. | kaolin | 2,00 |
| 7. | méthylphénylpolysiloxane (B-4) | 1,00 |
| 8. | polysaccharide (A-2) | 0,05 |
| 9. | phosphate d'acide ascorbique de magnésium | 3,00 |
| 10. | acide citrique | 1,00 |
| 11. | hydroxyde de sodium | quantité correcte |
| 12. | para-aminobenzoate de méthyle | 0,10 |
| 9. | eau de mer profonde | complément |

On prépare un mélange 3 en ajoutant les composants de mélange N° 4 à 7 aux composants de mélange N° 1 à 3 et en les agitant de façon à disperser de façon homogène les composants de mélange N° 4 à 7 dans les composants de mélange N° 1 à 3. De plus, on prépare un mélange 4 en agitant et en dissolvant les composants de mélange N° 8 à 13. On ajoute le mélange 3 au mélange 4 tout en agitant le mélange 4 pour les mélanger de façon homogène. On obtient ainsi une lotion 3 contenant 5,0 % de polysaccharide (A-2) par rapport à l'huile de silicone.

### [Préparation de la lotion 4]

On obtient la lotion 4 par le même procédé que pour la lotion 3, sauf que l'on remplace 0,05 % de polysaccharide A-2 en tant que composant de mélange N° 8 dans la lotion 3 par 0,05 % de gomme xanthane.

### [Préparation de la lotion 5]

On obtient la lotion 5 par le même procédé que pour la lotion 3, sauf que l'on remplace 1,00 % du méthylphénylpolysiloxane (B-4) en tant que composant de mélange N° 7 dans la lotion 3 par 1,00 % d'eau pure.

### [Préparation de la lotion laiteuse 1]

| (N°) | (Composants de mélange) | (% en poids) |
|---|---|---|
| 1. | méthylphénylpolysiloxane (B-4) | 10,00 |
| 2. | diméthylpolysiloxane (B-2) | 5,00 |
| 3. | diméthylpolysiloxane (B-3) | 5,00 |
| 4. | dioxyde de titane | 5,00 |
| 5. | oxyde de zinc | 3,00 |
| 6. | stéarate de sorbitan | 0,50 |
| 7. | acide stéarique | 0,50 |
| 8. | myristate de saccharose Dai-ichi (Cosmelike M-160, fabriqué par Kogyo Seiyaku Co., Ltd.) | 1,00 |
| 9. | polysaccharide (A-1) | 0,02 |
| 10. | glycérine | 3,00 |
| 11. | 1,3-butylèneglycol | 5,00 |
| 12. | para-oxybenzoate de méthyle | 0,10 |
| 13. | eau pure | complément |

On prépare un mélange 5 en agitant les composants de mélange N° 1 à 3, 6 et 7, tout en les chauffant à 70°C et en les agitant. De même, on prépare un mélange 6 en agitant les composants de mélange N° 8 à 13, tout en les chauffant à 70°C et en les agitant. On ajoute le mélange 5 au mélange 6 tout en agitant le mélange 6 à 5 000 tours par minute avec un homomélangeur pour préparer une émulsion. Ensuite, on ajoute les composants de mélange N° 4 et 5 à l'émulsion préparée, et on les mélange ensemble de façon homogène, et on les refroidit à la température ambiante tout en les agitant avec un agitateur à hélice, afin d'obtenir une lotion laiteuse 1 contenant 0,1 % de polysaccharide (A-1) par rapport à l'huile de silicone.

### [Préparation de la lotion laiteuse 2]

On obtient la lotion laiteuse 2 par le même procédé que pour la lotion laiteuse 1, sauf que l'on remplace 0,02 % de polysaccharide (A-1) en tant que composant de mélange N° 9 dans la lotion laiteuse 1 par 0,02 % de poly(acide acrylique).

### [Préparation de la lotion laiteuse 3]

On obtient la lotion laiteuse 3 par le même procédé que pour la lotion laiteuse 1, sauf que l'on remplace 0,02 % de polysaccharide (A-1) en tant que composant de mélange N° 9 dans la lotion laiteuse 1 par 0,01 % de polysaccharide (A-1) et 0,01 % d'eau pure, de façon à ce qu'elle contienne 0,05 % de polysaccharide (A-1) par rapport à l'huile de silicone.

### [Préparation de la lotion laiteuse 4]

On obtient la lotion laiteuse 4 par le même procédé que pour la lotion laiteuse 1, sauf que l'on remplace 0,02 % de polysaccharide (A-1) en tant que composant de mélange N° 9 et 0,13 % d'eau pure en tant que composant de mélange N° 14 dans la lotion laiteuse 1 par 0,15 % de gomme xanthane, on augmente la concentration du stéarate de sorbitan en tant que composant de mélange N° 6 de 0,50 % à 2,50 % et on augmente la concentration de myristate de saccharose en tant que composant de mélange N° 8 de 1,00 % à 7,50 %.

### [Préparation de la lotion laiteuse 5]

| (N°) | (Composants de mélange) | (% en poids) |
|---|---|---|
| 1. | méthylphénylpolysiloxane (B-4) | 8,00 |
| 2. | diméthylpolysiloxane (B-2) | 2,00 |
| 3. | dioxyde de titane | 5,00 |
| 4. | oxyde de zinc | 3,00 |
| 5. | oxyde de fer (rouge de fer) | 0,50 |
| 6. | oxyde de fer (jaune) | 1,50 |
| 7. | oxyde de fer (noir) | 0,20 |
| 8. | stéarate de sorbitan | 0,50 |
| 9. | acide stéarique | 0,50 |
| 10. | myristate de saccharose | 1,00 |
| 11. | polysaccharide (A-2) | 0,10 |
| 12. | glycérine | 3,00 |
| 13. | 1,3-butylèneglycol | 5,00 |
| 14. | para-oxybenzoate de méthyle | 0,10 |
| 15. | eau pure | complément |

On prépare un mélange 7 en agitant les composants de mélange N° 1 à 2, 8 et 9, tout en les chauffant à 70°C et en les agitant. De même, on prépare un mélange 8 en agitant les composants de mélange N° 10 à 15 à 70°C et en les agitant. On ajoute le mélange 7 au mélange 8 tout en agitant le mélange 8 à 5 000 tours par minute avec un homomélangeur pour préparer une émulsion. Ensuite, on ajoute les composants de mélange N° 3 à 7, et on les mélange ensemble de façon homogène, on les agite et on les refroidit à la température ambiante avec un agitateur à hélice, afin d'obtenir une lotion laiteuse 5 qui contient 1,0 % de polysaccharide (A-2) par rapport à l'huile de silicone.

### [Préparation de la lotion laiteuse 6]

On obtient la lotion laiteuse 6 par le même procédé que pour la lotion laiteuse 1, sauf que l'on remplace 0,02 % de polysaccharide (A-2) en tant que composant de mélange N° 9 dans la lotion laiteuse 5 par 0,02 % d'eau pure.

### [Préparation de la lotion laiteuse 7]

On obtient la lotion laiteuse 7 par le même procédé que pour la lotion laiteuse 4, sauf que l'on remplace 8,00 % de méthylphénylpolysiloxane (B-4) en tant que composant de mélange N° 1 et 2,00 % de diméthylpolysiloxane (B-2) en tant que composant de mélange N° 2 dans la lotion laiteuse 5 par 10,00 % d'eau pure.

### [Préparation de la lotion laiteuse 8] (ne faisant pas partie de la présente invention)

| (N°) | (Composants de mélange) | (% en poids) |
|---|---|---|
| 1. | monostéarate de sorbitan polyéthoxylé (10 moles d'addition) | 1,00 |
| 2. | trioléate de sorbitan polyéthoxylé (60 moles d'addition) | 0,50 |
| 3. | Monostéarate de glycéryle | 1,00 |
| 4. | Acide stéarique | 0,50 |
| 5. | diméthylpolysiloxane (B-2) | 5,00 |
| 6. | squalane | 4,00 |
| 7. | paraméthoxycinnamate d'isopropyle | 0,50 |
| 8. | polysaccharide (A-2) | 0,10 |
| 9. | phosphate d'acide ascorbique de magnésium | 5,00 |
| 10. | acide citrique | 1,50 |
| 11. | dioxyde de titane | 5,00 |
| 12. | pàraoxybenzoate de méthyle | 0,10 |
| 13. | polymère carboxyvinylique | 0,10 |
| 14. | hydroxyde de sodium | 0,05 |
| 15. | éthanol | 5,00 |
| 16. | eau pure | complément |

On prépare un mélange 9 en agitant et en mélangeant les composants de mélange N° 9 à 15 et la moitié du composant de mélange N° 16, et en maintenant la température à 70°C. On prépare un mélange 10 en chauffant et en agitant les composants de mélange N° 1 à 7 et en maintenant la température à 70°C. On ajoute le mélange 10 au mélange 9 tout en agitant le mélange 9 à 5 000 tours par minute avec un homomélangeur pour préparer une émulsion. On refroidit l'émulsion préparée à la température ambiante en l'agitant avec un agitateur à hélice. Ensuite, on dissout le polysaccharide (A-2) dans la moitié du composant N° 8 et on agite de façon homogène le mélange résultant avec l'émulsion préparée sous agitation, afin d'obtenir une lotion laiteuse 8 qui contient 2,0 % de polysaccharide (A-2) par rapport à l'huile de silicone.

### [Préparation de la lotion laiteuse 9]

On obtient la lotion laiteuse 9 par le même procédé que pour la lotion laiteuse 8, sauf que l'on remplace 0,10 % de polysaccharide (A-2) en tant que composant de mélange N° 8 et 0,20 % d'eau pure dans la lotion laiteuse 8 par 0,30 % de polysaccharide (A-2), pour qu'elle contienne 6,0 % de polysaccharide (A-2) par rapport à l'huile de silicone.

### [Préparation de la lotion laiteuse 10]

On obtient la lotion laiteuse 10 par le même procédé que pour la lotion laiteuse 8, sauf que l'on remplace 0,10 % de polysaccharide (A-2) en tant que composant de mélange N° 8 et 0,05 % d'eau pure en tant que composant de mélange N° 14 dans la lotion laiteuse 8 par 0,15 % de gomme xanthane.

### [Préparation de la crème 1]

| (N°) | (Composants de mélange) | (% en poids) |
|---|---|---|
| 1. | diméthylpolysiloxane (B-2) | 5,00 |
| 2. | monostéarate de glycéryle | 2,00 |
| 3. | acide stéarique | 2,00 |
| 4. | alcool stéarylique | 6,00 |
| 5. | lanoline hydrogénée | 4,00 |
| 6. | squalane | 9,00 |
| 7. | octyldodécanol | 10,00 |
| 8. | copolymère diméthylpolysiloxane-méthylsiloxane polyéthoxylé (B-6) | 5,00 |
| 9. | polysaccharide (A-2) | 0,05 |
| 10. | acide glycyrrhizinique | 0,20 |
| 11. | phosphate d'acide ascorbique de magnésium | 6,00 |
| 12. | acide citrique | 3,00 |
| 13. | hydroxyde de sodium | 0,20 |
| 14. | 1,3-butylèneglycol | 4,00 |
| 15. | para-oxybenzoate de méthyle | 0, 10 |
| 16. | eau pure | complément |

On prépare un mélange 11 en agitant les composants de mélange N° 1 à 7 et en les dissolvant par chauffage à 70 en les agitant. De même, on prépare un mélange 12 en dissolvant les composants de mélange N° 10 à 15 et la moitié du composant de mélange N° 16 en les chauffant. On ajoute le mélange 12 au mélange 11 tout en agitant le mélange 11 à 5 000 tours par minute avec un homomélangeur pour préparer une émulsion. On mélange une solution aqueuse préparée par dispersion et dissolution des composants de mélange N° 8 et 9 dans l'autre moitié du composant de mélange N° 16 avec l'émulsion préparée, tout en les agitant et on les refroidit à la température ambiante avec un agitateur à hélice, afin d'obtenir une crème 1 qui contient 1,0 % de polysaccharide (A-1) par rapport à l'huile de silicone.

### [Préparation de la crème 2]

On obtient la crème 2 par le même procédé que pour la crème 1, sauf que l'on remplace 0,05 % de polysaccharide (A-1) en tant que composant de mélange N° 9 et 0,15 % d'eau pure en tant que composant de mélange N° 16 dans la crème 1 par 0,20 % de gomme xanthane.

### [Préparation de la crème de fond de teint 1]

| (N°) | (Composants de mélange) | (% en poids) |
|---|---|---|
| 1. | lanoline | 7,00 |
| 2. | diméthylpolysiloxane (B-2) | 5,00 |
| 3. | diméthylpolysiloxane (B-3) | 2,00 |
| 4. | cétanol | 1,00 |
| 5. | paraméthoxycinnamate de 2-éthylhexyle | 3,00 |
| 6. | 4-t-butyl-4'-méthoxydibenzoylméthane | 1,00 |
| 7. | ester d'acide gras de sorbitan | 0,50 |
| 8. | glycérine | 5,00 |
| 9. | triéthanolamine | 1,00 |
| 10. | carboxyméthylcellulose (marque de commerce : CMC Daicel 1150, fabriquée par Daicel Chemical industries, Ltd.) | 0,10 |
| 11. | mica (marque de commerce : MICA Y3000, fabriqué par Yamaguchi-Mica Co., Ltd.) | 15,00 |
| 12. | talc (marque de commerce : Victory Light SK-A, fabriqué par Shokosan Laboratories Co.) Light | 6,00 |
| 13. | oxyde de fer | 6,50 |
| 14. | poudre de nylon (marque de commerce : Amilan, fabriquée par Toray Industries, Inc.) | 2,00 |
| 15. | silice (marque de commerce : Sun-sphere H-31, fabriquée par Asahi Glass Company) | 0,60 |
| 16. | polysaccharide (A-1) | 0,05 |
| 17. | Extrait de graines de coix lachryma jobi (fabriqué par Naruzen Pharmaceuticals Co., Ltd.) | 0,50 |
| 18. | phénoxyéthanol | 0,10 |
| 19. | eau pure | complément |

On prépare un mélange 13 en agitant les composants de mélange N° 1 à 7 pour les mélanger les uns avec les autres, et en les chauffant à 70 pour les dissoudre. De même, on prépare un mélange 14 en chauffant les composants de mélange N° 8 à 10, 16, 18 et 19 à 70°C pour les dissoudre. On ajoute le mélange 14 au mélange 13 tout en agitant le mélange 13 à 5 000 tours par minute avec un homomélangeur pour préparer une émulsion. On ajoute les composants de mélange N° 11 à 15 et 17 à l'émulsion préparée, tout en les agitant et on les refroidit à la température ambiante avec un agitateur à hélice, afin d'obtenir une crème de fond de teint 1 qui contient 0,7 % de polysaccharide (A-1) par rapport à l'huile de silicone.

### [Préparation de la crème de fond de teint 2]

On obtient la crème de fond de teint 2 par le même procédé que pour la crème de fond de teint 1, sauf que l'on remplace 0,05 % de polysaccharide (A-1) en tant que composant de mélange N° 16 dans la crème de fond de teint 1 par 0,05 % de gomme xanthane.

### [Préparation de la crème de fond de teint 2]

On obtient la crème de fond de teint 3 par le même procédé que pour la crème de fond de teint 1, sauf que l'on remplace 0,05 % de polysaccharide (A-1) en tant que composant de mélange N° 16 dans la crème de fond de teint 1 par 0,05 % de gomme xanthane et qu'on augmente l'ester d'acide gras de sorbitan en tant que composant de mélange N° 7 dans la crème de fond de teint 1 de 0,50 % à 3,00 %.

### [Préparation de la crème de fond de teint 4]

On obtient la crème de fond de teint 4 par le même procédé que pour la crème de fond de teint 1, sauf que l'on remplace 5,00 % de diméthylpolysiloxane (B-2) en tant que composant de mélange N° 2 et 2,00 % de diméthylpolysiloxane (B-3) en tant que composant de mélange N° 3 dans la crème de fond de teint 1 par 7,00 % d'eau pure.

### [Préparation du produit de rinçage capillaire 1] (ne faisant pas partie de la présente invention)

| (N°) | (Composants de mélange) | (% en poids) |
|---|---|---|
| 1. | chlorostéarate de triméthylammonium | 1,0 |
| 2. | cétanol | 3,0 |
| 3. | diméthylpolysiloxane (B-2) | 8,0 |
| 4. | stéaryléther polyéthoxylé (12 moles d'addition) | 1,0 |
| 5. | propylèneglycol | 5,0 |
| 6. | polysaccharide (A-1) | 0,1 |
| 7. | paraoxybenzoate de méthyle | 0,1 |
| 8. | chlorure de potassium | 0,3 |
| 9. | acide citrique | 0,2 |
| 10. | parfum | quantité correcte |
| 11. | eau pure | complément |

On prépare un mélange 15 en agitant les composants de mélange N° 1 à 5, et on prépare un mélange 16 en agitant les composants de mélange N° 6 à 11. Après avoir chauffé ces mélanges à 75°C, on ajoute lentement le mélange 15 au mélange 16 tout en agitant le mélange 16 avec un agitateur à hélice à quatre ailettes et en maintenant la température ci-dessus pour obtenir une émulsion. On agite encore l'émulsion et on la refroidit à la température ambiante pour obtenir un produit de rinçage capillaire 2 qui contient 1,25 % de polysaccharide (A-1) par rapport à l'huile de silicone.

### [Préparation du produit de rinçage capillaire 2]

On obtient le produit de rinçage capillaire 2 par le même procédé que pour le produit de rinçage capillaire 1, sauf que l'on remplace 0,10 % de polysaccharide (A-1) en tant que composant de mélange N° 6 et 0,10 % d'eau pure en tant que composant de mélange N° 11 dans le produit de rinçage capillaire 1 par 0,20 % de gomme xanthane.

### [Préparation de la poudre pour le visage 1]

| (N°) | (Composants de mélange) | (% en poids) |
|---|---|---|
| 1. | glycérine | 4,00 |
| 2. | 1,3-butylèneglycol | 4,00 |
| 3. | méthylphénylpolysiloxane | 5,00 |
| 4. | parfum | 0,01 |
| 5. | lauryléther polyéthoxylé (23 moles d'addition) | 0,20 |
| 6. | oxyde de fer (rouge) | 0,50 |
| 7. | oxyde de fer (jaune) | 1,40 |
| 8. | oxyde de fer (noir) | 0,20 |
| 9. | kaolin | 5,00 |
| 10. | dioxyde de titane | 5,00 |
| 11. | paraoxybenzoate de propyle | 0,10 |
| 12. | polysaccharide (A-2) | 0,06 |
| 13. | eau pure | complément |

On prépare un mélange 17 en agitant les composants de mélange N° 1 à 10, et on prépare un mélange 18 en agitant les composants de mélange N° 11 à 13. Après avoir chauffé ces mélanges à 75°C, on ajoute lentement le mélange 17 au mélange 18 tout en agitant le mélange 18 avec un agitateur à hélice à quatre ailettes et en maintenant la température ci-dessus pour obtenir une émulsion. On agite l'émulsion pour la refroidir à la température ambiante pour obtenir une poudre pour le visage 1 qui contient 1,2 % de polysaccharide (A-2) par rapport à l'huile de silicone.

### [Préparation de la poudre pour le visage 2]

On prépare la poudre pour le visage 2 par le même procédé que pour la poudre pour le visage 1, sauf que l'on remplace 5,00 % de méthylphénylpolysiloxane (B-4) en tant que composant de mélange N° 3 dans la poudre pour le visage 1 par 5,00 % d'eau pure.

### [Préparation de la poudre pour le visage 3]

On prépare la poudre pour le visage 3 par le même procédé que pour la poudre pour le visage 1, sauf que l'on remplace 0,06 % de polysaccharide (A-2) en tant que composant de mélange N° 12 et 5,00 % de méthylphénylpolysiloxane (B-4) en tant que composant de mélange N° 3 dans la poudre pour le visage 1 par 5,06 % d'eau pure.

### [Préparation du crayon pour les yeux 1]

| (N°) | (Composants de mélange) | (% en poids) |
|---|---|---|
| 1. | oxyde de fer (noir) | 4,00 |
| 2. | dioxyde de titane | 5,00 |
| 3. | pigment perlé | 0,20 |
| 4. | éthanol | 4,00 |
| 5. | glycérine | 6,00 |
| 6. | huile de ricin hydrogénée polyéthoxylée (100 moles d'addition | 0,50 |
| 7. | diméthylpolysiloxane (B-1) | 10,10 |
| 8. | polysaccharide (A-1) | 0,08 |
| 9. | phénoxyéthanol | 0,15 |
| 13. | eau pure | complément |

On prépare un mélange 19 en dispersant de façon homogène les composants de mélange N° l à 3 dans les composants de mélange 4 à 7. On prépare un mélange 20 en agitant et en dissolvant les composants de mélange N° 8 et 9. On ajoute le mélange 19 au mélange 20 tout en agitant le mélange 20 de façon homogène pour obtenir le crayon pour les yeux 1 contenant 0,8 % de polysaccharide (A-1) par rapport à l'huile de silicone.

### [Préparation du crayon pour les yeux 2]

On obtient le crayon pour les yeux 2 par le même procédé que pour le crayon pour les yeux 1, sauf que l'on remplace 10,00 % de diméthylpolysiloxane (B-1) en tant que composant de mélange N° 7 dans le crayon pour les yeux 1 par 10,00 % d'eau pure.

### [Préparation du crayon pour les yeux 3]

On obtient le crayon pour les yeux 3 par le même procédé que pour le crayon pour les yeux 1, sauf que l'on remplace 10,00 % de diméthylpolysiloxane (B-1) en tant que composant de mélange N° 7 et 0,08 % de polysaccharide (A-1) en tant que composant de mélange N° 8 dans le crayon pour les yeux 1 par 10,08 % d'eau pure.

### [Préparation du rouge pour les joues 1]

| (N°) | (Composants de mélange) | (% en poids) |
|---|---|---|
| 1. | kaolin | 10,00 |
| 2. | dioxyde de titane | 2,00 |
| 3. | oxyde de fer (rouge) | 0,20 |
| 4. | rouge N° 202 | 0,30 |
| 5. | éthanol | 4,00 |
| 6. | glycérine | 4,00 |
| 7. | ester d'acide gras de polyglycérine | 0,50 |
| 8. | méthylphénylpolysiloxane (B-4) | 5,00 |
| 9. | parfum | 0,02 |
| 8. | polysaccharide (A-2) | 0,10 |
| 9. | phénoxyéthanol | 0,15 |
| 13. | eau pure | complément |

On prépare un mélange 21 en dispersant de façon homogène les composants de mélange N° 1 à 4 dans les composants de mélange 5 à 9 et dans une partie du composant de mélange N° 12. On prépare un mélange 22 en agitant et en dissolvant les composants de mélange N° 10 à 12. On ajoute le mélange 21 au mélange 22 tout en agitant le mélange 22 de façon homogène pour obtenir le rouge pour les joues 1 contenant 2,0 % de polysaccharide (A-2) par rapport à l'huile de silicone.

### [Préparation du rouge pour les joues 2]

On obtient le rouge pour les joues 2 par le même procédé que pour le rouge pour les joues 1, sauf que l'on remplace 5,00 % de méthylphénylpolysiloxane (B-4) en tant que composant de mélange N° 8 dans le rouge pour les joues 1 par 5,00 % d'eau pure.

### [Préparation du rouge pour les joues 3]

On obtient le rouge pour les joues 3 par le même procédé que pour le rouge pour les joues 1, sauf que l'on remplace 5,00 % de méthylphénylpolysiloxane (B-4) en tant que composant de mélange N° 8 et 0,10 % de polysaccharide (A-2) en tant que composant de mélange N° 10 dans le rouge pour les joues 1 par 5,10 % d'eau pure.

### [Préparation d'une crème en émulsion composite E/H/E] (ne faisant pas partie de la présente invention)

| (N°) | (Composants de mélange) | (% en poids) |
|---|---|---|
| 1. | monostéarate de polyéthoxylé (10 moles d'addition) | 2,00 |
| 2. | monostéarate de glycérine | 5,00 |
| 3. | diméthylpolysiloxane (B-1) | 1,50 |
| 4. | acide stéarique | 5,00 |
| 5. | squalane | 15,00 |
| 6. | isocyanate de cétyle | 5,00 |
| 7. | paraoxybenzoate de méthyle | 0,10 |
| 8. | 1,3-butylèneglycol | 5,00 |
| 9. | indométhacine | 0,50 |
| 10. | acide hyaluronique | 0,01 |
| 11. | préparation de parfum à base de menthe | 0,001 |
| 12. | polysaccharide (A-2) | 0,05 |
| 13. | dioxyde de titane | 2,00 |
| 14. | eau pure | complément |

On prépare un mélange 23 en chauffant et en agitant les composants de mélange N° 1 à 3 et en maintenant la température à 70°C. On prépare un mélange 24 en agitant les composants de mélange N° 7 à 11 avec une partie du composant de mélange N° 14, et en maintenant la température à 70°C. On ajoute le mélange 24 au mélange 23 tout en agitant le mélange 23 avec un homomélangeur de petite taille pour préparer une émulsion E/H. On prépare un mélange 25 en chauffant et en agitant les composants de mélange N° 4 à 6 et en maintenant la température à 70°C. On ajoute successivement les mélanges N° 23, 25 et le composant de mélange N° 13 aux composants de mélange N° 12 et 14 en les agitant et en mélangeant les composants de mélange N° 12 et 14 de façon homogène pour obtenir une crème en émulsion E/H/E contenant 3,3 % de polysaccharide (A-2) par rapport à l'huile de silicone.

### [Préparation d'une crème en microcapsule H/E/H]

| (N°) | (Composants de mélange) | (% en poids) |
|---|---|---|
| 1. | diméthylpolysiloxane (B-1) | 5,00 |
| 2. | sabacate de dioctyle | 15,00 |
| 3. | 1,3-butylèneglycol | 10,00 |
| 4. | huile de ricin hydrogénée polyéthoxylée 3 (100 moles d'addition) | 1,00 |
| 5. | indométhacine | 0,50 |
| 6. | polysaccharide (A-1) | 0,04 |
| 7. | gomme agar | 0,80 |
| 8. | eau pure | complément |
| 9. | dioxyde de titane | 2,00 |
| 10. | copolymère polyoxyéthylène-polysiloxane | 1,00 |
| 11. | octaméthylcyclotétrasiloxane | 49,00 |

On prépare un mélange 26 en agitant les composants de mélange N° 3 à 5 de façon homogène. On prépare un mélange 27 en agitant les composants de mélange N° 1 et 2 de façon homogène. On ajoute lentement le mélange 26 au mélange 27 pour préparer un mélange 28. On chauffe et on agite les composants de mélange N° 6, 7 et 8 les uns avec les autres, et on maintient la température à 50°C pour préparer un mélange 29. Et, on ajoute le mélange 28 au mélange 29 tout en agitant le mélange 29 pour préparer des microcapsules H/E. On mélange de façon homogène les composants de mélange N° 9 à 11, et on y ajoute les microcapsules H/E, afin d'obtenir une crème en microcapsule H/E/H contenant 0,67 % de polysaccharide (A-1) par rapport à l'huile de silicone.

### [Préparation de la lotion laiteuse 11 (nano-émulsion)] (ne faisant pas partie de la présente invention)

| (N°) | (Composants de mélange) | (% en poids) |
|---|---|---|
| 1. | monostéarate de sorbitan polyéthoxylé (10 EO) | 1,00 |
| 2. | tétraoléate de sorbitan polyéthoxylé 60 EO) | 0,50 |
| 3. | monostéarate de glycérine | 1,00 |
| 4. | diméthylpolysiloxane (B-1) | 2,00 |
| 5. | alcool bénénylique | 0,50 |
| 6. | squalane | 8,00 |
| 7. | paraméthoxycinnamate d'isopropyle | 0,50 |
| 8. | polysaccharide (A-1) | 0,10 |
| 9. | phosphate d'acide ascorbique de magnésium | 5,00 |
| 10. | acide citrique | 1,50 |
| 11. | dioxyde de titane | 5,00 |
| 12. | paraoxybenzoate de méthyle | 0,10 |
| 13. | polymère carbovinylique | 0,10 |
| 14. | hydroxyde de sodium | 0,05 |
| 15. | éthanol | 5,00 |
| 16. | huile d'orange | 0,05 |
| 17. | eau pure | complément |

On prépare un mélange 30 en chauffant les composants de mélange N° 9 à 15 et une partie du composant de mélange 17, en les mélangeant les uns avec les autres et en maintenant la température à 70°C. Après avoir chauffé et mélangé les composants de mélange N° 1 à 7 et le composant de mélange N° 16, on ajoute le mélange résultant au mélange 30, et on les agite ensemble en utilisant un turbomélangeur-homogénéisateur tout en maintenant la température à 70°C. Ensuite, on ajoute une solution aqueuse de polysaccharide (A-1) en tant que composant de mélange N° 8 en utilisant un homogénéisateur haute pression (MODEL 3MR fabriqué par APVGAULIN Inc.) sous une pression de 1 200 bars tout en maintenant la température du produit à environ 35°C ou moins, et on agite de façon homogène le mélange obtenu, afin d'obtenir une nano-émulsion qui contient 5,0 % de polysaccharide (A-1) par rapport à l'huile de silicone.

### [Essai de stabilité de la composition cosmétique mélangée avec une huile de silicone]

Après avoir prélevé des échantillons de 200 ml de la composition cosmétique mélangée avec une huile de silicone, juste après sa préparation, dans une bouteille d'échantillon et en mesurant la viscosité de la composition, on ferme hermétiquement la bouteille et on la place dans une enceinte thermostatée à 45°C. Ensuite, on mesure de nouveau la viscosité au bout de 12 semaines. On prélève un échantillon de 100 ml de la composition cosmétique mélangée avec une huile de silicone dans un cylindre de mesure de 100 ml, et on ferme hermétiquement le cylindre et on le place dans une enceinte thermostatée à 45°C. Au bout de 12 semaines, on mesure la volume de la couche d'huile de silicone flottant sur la composition cosmétique dans le cylindre de mesure de 100 ml, et le volume de la phase aqueuse séparée dans la composition cosmétique. Les résultats évalués par les critères d'évaluation suivants sont présentés dans le tableau 1.

### (Critères d'évaluation de la stabilité)

○ : pas de séparation ni de précipitation observé à l'oeil ; et
X : séparation et précipitation observés à l'oeil.

### (Evaluation de la sensation lors d'utilisations)

On divise respectivement la composition cosmétique mélangée avec un huile de silicone (A) présentée dans le tableau 1, juste après sa préparation, et la composition cosmétique mélangée avec une huile de silicone (B) après avoir été placée dans l'enceinte thermostatique à 45°C pendant 12 semaines continues, en dix jeux de récipients ayant chacun un aspect externe identique les uns aux autres de sorte qu'ils ne peuvent pas être distingués les uns des autres. On chiosit deux experts dans chaque tranche d'âge de dix à cinquante ans. Et dix experts au total réalisent les essais sensoriels. On étale une quantité correcte de chaque composition cosmétique mélangée avec une huile de silicone (A), hormis les produits de rinçage capillaires 1 et 2, sur le dos des deux mains de chaque expert, et on évalue respectivement "le caractère collant" et "l'uniformité" de celle-ci. On évalue aussi la composition cosmétique mélangée avec une huile de silicone (B) de la même manière. On évalue le produit de rinçage capillaire par la sensation lorsque chaque expert touche leur cheveux après avoir utilisé le produit de rinçage capillaire. On évalue l'échantillon présentant une précipitation et une séparation après agitation. Les critères de "caractère collant" et "d'uniformité" de la manière suivante. Les résultats sont présentés dans le tableau 2.

### (Critère d'évaluation du "caractère collant")

○ : évalué par huit des dix experts ou plus comme ayant une sensation fraîche avec peu de sensation collante.
Δ : évalué par cinq à sept des dix experts comme ayant une sensation fraîche avec peu de sensation collante.
X : évalué par quatre des dix experts ou moins comme ayant une sensation fraîche avec peu de sensation collante

### (Evaluation du critère "d'uniformité"

○ : évalué par huit des dix experts ou plus comme ayant une sensation lisse.
Δ : évalué par cinq à sept des dix experts comme ayant une sensation lisse.
X : évalué par quatre des dix experts ou moins comme ayant une sensation lisse.

On n'observe aucune séparation ni aucun flottement de l'huile de silicone, ni une quelconque séparation de l'émulsion dans la composition cosmétique mélangée avec une huile de silicone préparée par le procédé de la présente invention après un stockage pendant une durée prolongée, et on a ainsi prouvé que les produits cosmétiques constitués de la composition cosmétique mélangée avec une huile de silicone préparée par le procédé de la présente invention présentent une excellente sensation à l'emploi.

### Avantages de l'invention

Comme décrit ci-dessus, avec le procédé selon la présente invention, on empêche la séparation de l'huile de silicone ainsi que des produits cosmétiques, et on empêche une augmentation de la viscosité des produits cosmétiques après un stockage de la composition cosmétique mélangée avec une huile de silicone pendant une durée prolongée, améliorant de ce fait la stabilité de l'émulsion et, par conséquent, contribuant grandement à l'amélioration des qualités des produits cosmétiques. Et les produits cosmétiques obtenus par le procédé de la présente invention présentent une sensation améliorée à l'emploi, qui n'a pas été rencontrée avec les produits cosmétiques classiques, améliorant de ce fait les qualités de ceux-ci. Par conséquent, le procédé de la présente invention convient comme procédé de stabilisation de la composition cosmétique mélangée avec une huile de silicone.

**TABLEAU 1**

| Example | Composition cosmétique mélangée avec une huile de silicone | Viscosité (mPa.s) | | Séparation et précipitation |
|---|---|---|---|---|
| | | Immédiatement après préparation | au bout de 12 semaines | |
| | lotion 1 ^{a)} | 45 | 40 | ○ |
| | lotion 3 ^{a)} | 105 | 105 | ○ |
| | lotion laiteuse 1 | 150 | 145 | ○ |
| | lotion laiteuse 5 | 265 | 270 | ○ |
| Modes de | lotion laiteuse 8 ^{a)} | 315 | 310 | ○ |
| réalisation | crème 1 | 750 | 750 | ○ |
| | fond de teint | 3265 | 3260 | ○ |
| | produit de rinçage ^{a)} capillaire 1 | 465 | 460 | ○ |
| | poudre pour le visage 1 | 370 | 370 | ○ |
| | crayon pour les yeux 1 | 430 | 435 | ○ |
| | rouge pour les joues 1 | 525 | 525 | ○ |
| | crème en émulsion composite E/H/E ^{a)} | 305 | 300 | ○ |
| | crème en microcapsule H/B/H | 460 | 450 | ○ |
| | préparation d'une lotion laiteuse en nano-émulaion lotion laiteuse 1 ^{a)} | 325 | 320 | ○ |

| | | | | |
|---|---|---|---|---|
| a) ne faisant pas partie de la présente invention | | | | |

**TABLEAU 2**

| Exemple | Composition cosmétique mélangée avec une huile de silicone | viscosité (mPa·s) | | Séparation et précipitation |
|---|---|---|---|---|
| | | Immédiatement après préparation | au bout de 12 semaines | |
| | lotion 2 | 65 | 30 | X |
| Exemples | lotion 4 | 50 | 25 | X |
| comparatifs | lotion 5 | 100 | 95 | ○ |
| | lotion laiteuse 2 | 165 | 120 | X |
| | lotion laiteuse 3 | 145 | 105 | X |
| | lotion laiteuse 4 | 150 | 115 | X |
| | lotion laiteuse 6 | 280 | 385 | X |
| | lotion laiteuse 7 | 260 | 265 | ○ |
| | lotion laiteuse 9 | 765 | 985 | ○ |
| | lotion laiteuse 10 | 325 | 520 | X |
| | crème 2 | 650 | 1020 | X |
| | fond de teint 2 | (*) | (*) | X |
| | fond de teint 3 | (*) | (*) | X |
| | fond de teint 4 | 3215 | 3220 | ○ |
| | Produit de rinçage capillaire 2 | 430 | 875 | X |
| | poudre pour le visage 2 | 345 | 340 | ○ |
| | poudre pour le visage 3 | (*) | (*) | X |
| | crayon pour les yeux 2 | 410 | 405 | ○ |
| | crayon pour les yeux 3 | (*) | (*) | X |
| | rouge pour les joues 2 | 530 | 530 | ○ |
| | rouge pour les joues 3 | (*) | (*) | X |

| | | | | |
|---|---|---|---|---|
| (*) Non mesurable en raison d'une séparation et d'une précipitation. | | | | |

**TABLEAU 3**

| Exemple | Composition cosmétique mélangée avec une huile de silicone | caractère collant | | Uniformité | |
|---|---|---|---|---|---|
| | | juste après préparation | au bout de 12 semaines | juste après préparation | au bout de 12 semaines |
| Modes de réalisation | lotion 1 ^{a)} | ○ | ○ | ○ | ○ |
| | lotion 3 ^{a)} | ○ | ○ | ○ | ○ |
| | lotion laiteuse 1 | ○ | ○ | ○ | ○ |
| | lotion laiteuse 5 | ○ | ○ | ○ | ○ |
| | lotion laiteuse 8 ^{a)} | ○ | ○ | ○ | ○ |
| | crème 1 | ○ | ○ | ○ | ○ |
| | fond de teint | ○ | ○ | ○ | ○ |
| | produit de rinçage ^{a)} capillaire 1 | ○ | ○ | ○ | ○ |
| | poudre pour le visage 1 | ○ | ○ | ○ | ○ |
| | crayon pour les yeux 1 | ○ | ○ | ○ | ○ |
| | rouge pour les joues 1 | ○ | ○ | ○ | ○ |
| | crème en émulsion composite E/H/E ^{a)} | ○ | ○ | ○ | ○ |
| | crème en microcapsule H/E/H | ○ | ○ | ○ | ○ |
| | préparation d'une lotion laiteuse en nana-émulsion lotion laiteuse 1 ^{a)} | ○ | ○ | ○ | ○ |

| | | | | | |
|---|---|---|---|---|---|
| a) ne faisant pas partie de la présente invention | | | | | |

**TABLEAU 4**

| Exemple | Composition cosmétique mélangée avec une huile de silicone | caractère collant | | Uniformité | |
|---|---|---|---|---|---|
| | | juste après préparation | au bout de 12 semaines | juste après préparation | au bout de 12 semaines |
| | lotion 2 | x | x | x | x |
| | lotion 4 | x | x | x | x |
| | lotion 5 | ○ | ○ | Δ | ○ |
| | lotion laiteuse 2 | x | x | x | x |
| | lotion laiteuse 3 | x | x | x | x |
| | lotion laiteuse 4 | x | x | x | x |
| | lotion laiteuse 6 | x | x | x | x |
| | lotion laiteuse 7 | ○ | ○ | Δ | ○ |
| Exemples | lotion laiteuse 9 | Δ | Δ | x | ○ |
| comparatifs | lotion laiteuse 10 | x | x | x | x |
| | crème 2 | x | x | x | x |
| | fond de teint 2 | x | x | x | x |
| | fond de teint 3 | x | x | x | x |
| | fond de teint 4 | ○ | ○ | x | ○ |
| | Produit de rinçage | x | x | x | x |
| | capillaire 2 poudre pour le visage 2 | ○ | ○ | x | ○ |
| | poudre pour le visage 3 | x | x | x | x |
| | crayon pour les yeux 2 | ○ | ○ | x | ○ |
| | crayon pour les yeux 3 | x | x | x | x |
| | rouge pour les joues 2 | ○ | ○ | x | ○ |
| | rouge pour les joues 3 | x | x | x | x |

## Revendications

1. Procédé de stabilisation d'une composition cosmétique émulsifiée-mélangée avec une huile de silicone comprenant des étapes de mélange d'un polysaccharide constitué au moins de fucose, de glucose, d'acide glucuronique et de rhamnose en tant que monosaccharides constitutifs dans la composition cosmétique émulsifiée et de mélange d'un tensioactif dans la composition cosmétique émulsifiée de sorte que la teneur dudit tensioactif s'échelonne de 0,1 à 20 % en poids, par rapport à l'huile de silicone.

2. Procédé de stabilisation d'une composition cosmétique émulsifiée mélangée avec une huile de silicone selon la revendication 1, dans lequel ledit polysaccharide est un produit d'un micro-organisme de la souche Alcaligenes latus B-16

3. Procédé de stabilisation d'une composition cosmétique émulsifiée mélangée avec une huile de silicone selon la revendication 1 ou 2, dans lequel ledit tensioactif est au moins un composé parmi un ester d'acide gras de saccharose, un ester d'acide gras de glycérine, un ester d'acide gras de polyglycérine, la lécithine et les dérivés de ceux-ci.

4. Procédé de stabilisation d'une composition cosmétique émulsifiée mélangée avec une huile de silicone selon la revendication 1, 2 ou 3, dans lequel la teneur dudit polysaccharide s'échelonne de 0,1 à 5 % en poids, convertie en fraction solide séchée, par rapport à l'huile de silicone.

## Claims

1. A method for stabilizing an emulsified cosmetic composition blended with silicone oil comprising steps of blending a polysaccharide composed of at least fucose, glucose, glucuronic acid and rhamnose as constituting monosaccharides in the emulsified cosmetic composition and blending a surfactant in the emulsified cosmetic composition such that the content of said surfactant ranges from 0.1 to 20 % by weight, with respect to silicone oil.

2. The method for stabilizing an emulsified cosmetic composition blended with silicone oil according to claim 1, wherein said polysaccharide is a product of a microorganism of Alcaligenes latus strain B-16.

3. The method for stabilizing an emulsified cosmetic composition blended with silicone oil according to claim 1 or 2, wherein said surfactant is at least one of sucrose fatty acid ester, glycerin fatty acid ester, polyglycerin fatty acid ester, lecithin and derivatives thereof.

4. The method for stabilizing an emulsified cosmetic composition blended with silicone oil according to claim 1, 2 or 3, wherein the content of said polysaccharide ranges from 0.1 to 5 % by weight as converted into a dried solid fraction with respect to silicone oil.

## Patentansprüche

1. Verfahren zur Stabilisierung einer, mit einem Silikonöl gemischten kosmetischen Emulsionszusammensetzung, die die Verfahrensschritte des Vermischens eines Polysaccharids aus zumindest einem von Fucose, Glucose, Glucuronsäure und Rhamnose als konstituierende Monosaccharide in der kosmetischen Emulsionszusammensetzung, und des Einmischens eines Tensids in die kosmetische Emulsionszusammenstzung, sodass sich der Gehalt des Tensids bezüglich des Silikonölgehalts von 0,1 bis 2,0 Gew% erstreckt.

2. Verfahren zur Stabilisierung einer, mit einem Silikonöl gemischten, kosmetischen Emulsionszusammensetzung nach Anspruch 1, in der das Polysaccharid ein Produkt eines Mikroorganismus des Stammes Alcaligenes latus B-16 ist.

3. Verfahren zur Stabilisierung einer, mit einem Silikonöl gemischten kosmetischen Emulsionszusammensetzung nach Anspruch 1 oder 2, in der das Tensid zumindest eine Verbindung unter Saccharose Fettsäureester, Glyzerin Fettsäureester, Polyglyzerin Fettsäureester, Lecithin und Abkömmlingen hiervon ist.

4. Verfahren zur Stabilisierung einer, mit einem Silikonöl gemischten kosmetischen Emulsionszusammensetzung nach Anspruch 1, 2 oder 3, in dem der Gehalt des Polysaccharids bezüglich des Silikonöls und in trockene feste Phase konvertiert, sich von 0,1 bis 5 Gew% erstreckt.
